# EUROPÄISCHE PATENTSCHRIFT

(11) **EP 0 757 673 B1**
(45) Veröffentlichungstag und Bekanntmachung des Hinweises auf die Patenterteilung: **04.10.2001**
(21) Anmeldenummer: 95916668.7
(22) Anmeldetag: 18.04.1995
(51) Int. Cl.: C07D 209/48, C07D 263/38, C07D 263/44, C07D 211/88, C07D 207/448, C07D 209/46, C07D 249/20, C07D 249/16, C07C 311/08, A01N 43/38

(54) **HETEROCYCLYLBENZONITRILE**
HETEROCYCLYLBENZONITRILES
HETEROCYCLYLBENZONITRILES

(30) Priorität: 27.04.1994 DE 4414568
(43) Veröffentlichungstag der Anmeldung: 12.02.1997
(73) Patentinhaber: BAYER AG, 51368 Leverkusen (DE)
(72) Erfinder: DREWES, Mark-Wilhelm, D-40764 Langenfeld (DE); ANDREE, Roland, D-40764 Langenfeld (DE); FINDEISEN, Kurt, D-51375 Leverkusen (DE); HAAS, Wilhelm, D-50259 Pulheim (DE); LENDER, Andreas, D-21376 Salzhausen (DE); LINKER, Karl-Heinz, D-51377 Leverkusen (DE); SCHALLNER, Otto, D-40789 Monheim (DE); DOLLINGER, Markus, D-51381 Leverkusen (DE); SANTEL, Hans-Joachim, D-51371 Leverkusen (DE)
(86) Internationale Anmeldenummer: EP9501441
(87) Internationale Veröffentlichungsnummer: WO9529158

(56) Entgegenhaltungen:
- EP-A- 0 364 797
- EP-A- 0 370 332
- EP-A- 0 558 999
- EP-A- 0 648 772

## Beschreibung

Die Erfindung betrifft neue Heterocyclylbenzonitrile, Verfahren und neue Zwischenprodukte zu ihrer Herstellung und ihre Verwendung als Herbizide.

Es ist bereits bekannt, dass bestimmte Heterocyclylbenzonitrile, wie z.B. die Verbindungen 2,5-Difluor-4-(4,5,6,7-tetrahydro-3-methyl-2H-indazol-2-yl)-benzonitril und 4-[5-(t-Butyl)-2-oxo-1,3,4-oxadiazol-3(2H)-yl]-2,5-difluor-benzonitril herbizide Eigenschaften aufweisen (vgl. EP-A 370332, Beispiele 2 und 7; vgl. auch EP-A 364797 und EP-A 558999). Die Wirksamkeit dieser vorbekannten Verbindungen ist jedoch, insbesondere bei niedrigen Aufwandmengen und Konzentrationen nicht in allen Anwendungsgebieten völlig zufriedenstellend.

Es wurden nun die neuen Heterocyclylbenzonitrile der allgemeinen Formel (I) gefunden, 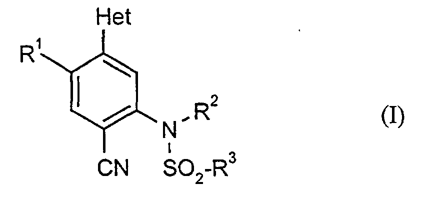 in welcher
- R¹: für Wasserstoff oder Halogen steht,
- R²: für Wasserstoff, für Formyl oder für jeweils gegebenenfalls durch Halogen, Cyano, C₁-C₄-Alkoxy oder C₁-C₄-Alkoxy-carbonyl substituiertes Alkyl, Alkenyl, Alkinyl, Alkoxy, Alkylcarbonyl, Alkoxycarbonyl oder Alkylsulfonyl mit jeweils bis zu 6 Kohlenstoffatomen, für jeweils gegebenenfalls durch Halogen, Cyano oder C₁-C₄-Alkyl substituiertes Cycloalkyl, Cycloalkylalkyl, Cycloalkylcarbonyl oder Cycloalkylsulfonyl mit 3 bis 6 Kohlenstoffatomen im Cycloalkylteil und gegebenenfalls 1 bis 4 Kohlenstoffatomen im Alkylteil, oder für Phenylmethyl, Phenylcarbonyl, Naphthylcarbonyl, Phenylmethylcarbonyl, Phenoxycarbonyl, Phenylsulfonyl, Naphthylsulfonyl, Phenylmethylsulfonyl, Thienylsulfonyl, Pyrazolylsulfonyl, Pyridinylsulfonyl oder Pyridinylmethylsulfonyl (welche jeweils gegebenenfalls durch Halogen, Cyano, C₁-C₄-Alkyl, C₁-C₄-Halogenalkyl, C₁-C₄-Alkoxy, C₁-C₄-Halogenalkoxy oder C₁-C₄-Alkoxy-carbonyl substituiert sind) steht,
- R³: für jeweils gegebenenfalls durch Halogen, Cyano oder C₁-C₄-Alkoxy substituiertes Alkyl, Alkenyl oder Alkinyl mit jeweils bis zu 10 Kohlenstoffatomen steht, oder für jeweils gegebenenfalls durch Halogen, Cyano oder C₁-C₄-Alkyl substituiertes Cycloalkyl oder Cycloalkylalkyl mit 3 bis 8 Kohlenstoffatomen im Cycloalkylteil und gegebenenfalls 1 bis 4 Kohlenstoffatomen im Alkylteil steht, oder für jeweils gegebenenfalls durch Halogen, Cyano, Nitro, Carboxy, Carbamoyl, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Fluor und/oder Chlor substituiert sind), durch Dimethylaminosulfonyl, Diethylaminosulfonyl, Dimethylaminocarbonyl oder Diethylaminocarbonyl, durch C₁-C₄-Alkoxy-carbonyl (welches gegebenenfalls durch Halogen, Methoxy oder Ethoxy substituiert ist), durch Phenyl, Phenyloxy oder Phenylthio (welche jeweils gegebenenfalls durch Halogen, Cyano, Methyl, Methoxy, Trifluormethyl und/oder Trifluormethoxy substituiert sind) substituiertes Aryl oder Arylalkyl mit 6 oder 10 Kohlenstoffatomen im Arylteil und 1 bis 4 Kohlenstoffatomen im Alkylteil, oder für jeweils gegebenenfalls durch Halogen, Cyano, Nitro, Carboxy, Carbamoyl, durch C₁-C₄-Alkyl, C₁-C₄-Alkoxy, C₁-C₄-Alkylthio, C₁-C₄-Alkylsulfinyl, C₁-C₄-Alkylsulfonyl oder C₁-C₄-Alkoxycarbonyl (welche jeweils gegebenenfalls durch Halogen substituiert sind), durch Phenyl, Phenoxy oder Phenylthio (welche jeweils gegebenenfalls durch Halogen, Cyano, C₁-C₄-Alkyl, C₁-C₄-Halogenalkyl, C₁-C₄-Alkoxy und/oder C₁-C₄-Halogenalkoxy substituiert sind) substituiertes Heterocyclyl oder Heterocyclylalkyl mit 2 bis 6 Kohlenstoffatomen und 1 bis 4 Stickstoffatomen und/oder 1 bis 2 Sauerstoffoder Schwefelatomen im gesättigten oder ungesättigten Heterocyclylteil und gegebenenfalls 1 bis 4 Kohlenstoffatomen im Alkylteil steht, und
- Het: für eine der im folgenden aufgeführten (über N gebundenen) heterocyclischen Gruppierungen steht, 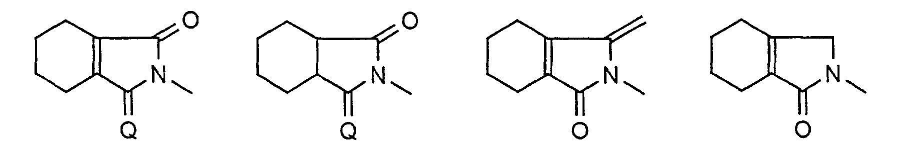 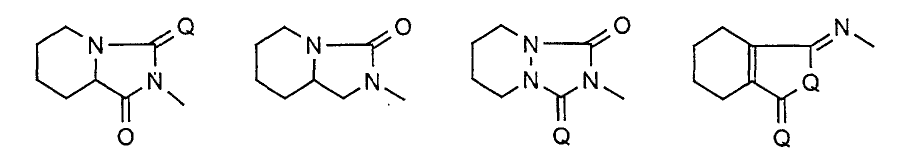 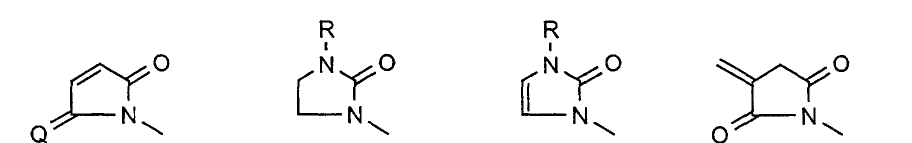 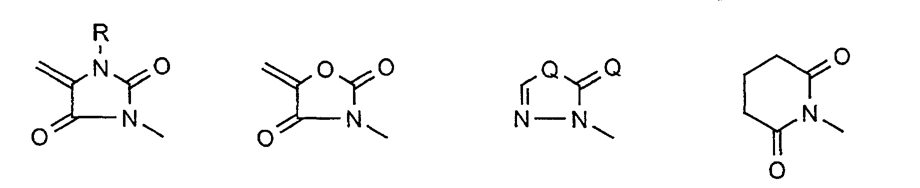 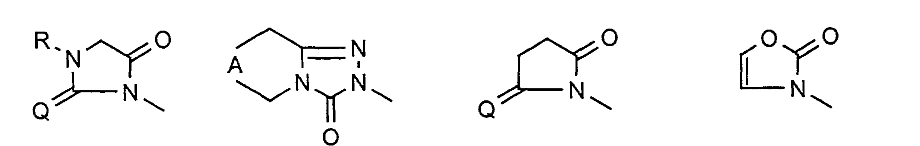 worin jeweils gegebenenfalls
- A: für gegebenenfalls durch SO₂ unterbrochenes C₁-C₃-Alkandiyl steht und
- Q: für Sauerstoff oder Schwefel steht und wobei die angegebenen heterocyclischen Gruppierungen jeweils gegebenenfalls einfach bis dreifach substituiert sind durch gleiche oder verschiedene Reste aus der Reihe Hydroxy, Halogen, Cyano, Nitro, C₁-C₄-Alkyl, C₁-C₄-Halogenalkyl, C₁-C₄-Alkoxy, C₁-C₄-Halogenalkoxy oder Phenyl und
- R: für einen Rest aus der Reihe Wasserstoff, Hydroxy, Cyano, Nitro, C₁C₄-Alkyl, C₁-C₄-Halogenalkyl, C₁-C₄-Alkoxy, C₁-C₄-Halogenalkoxy oder Phenyl steht. Weiter wurde gefunden, dass man Heterocyclylbenzonitrile der allgemeinen Formel (I) erhält, wenn man
(a) Anhydride der allgemeinen Formeln (IIa) bis (IIg), 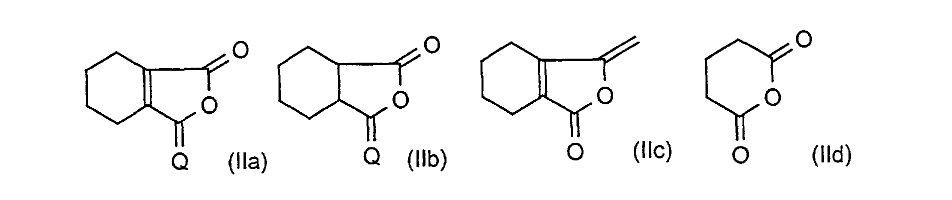 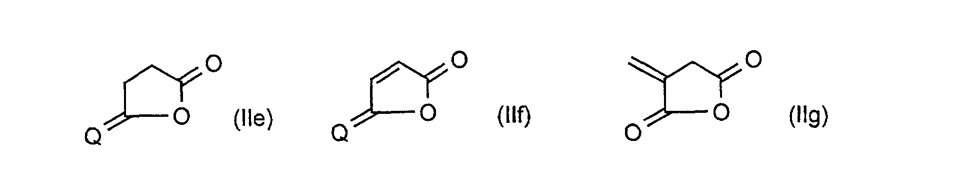 in welcher
- Q: die in Anspruch 1 oder 2 angegebene Bedeutung hat,
mit Aminobenzonitrilen der allgemeinen Formel (III) 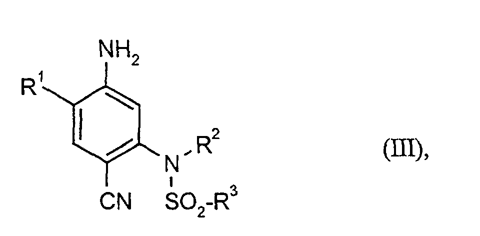 in welcher
R¹, R² und R³ die in Anspruch 1 oder 2 angegebenen Bedeutungen haben,
gegebenenfalls in Gegenwart eines Verdünnungsmittels und gegebenenfalls in Gegenwart eines Reaktionshilfsmittels umsetzt,
oder dass man
(b) halogenierte Heterocyclylbenzonitrile der allgemeinen Formel (IV) 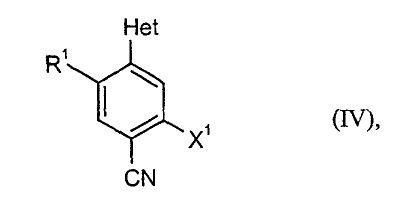 in welcher
Het und R¹ die in Anspruch 1 oder 2 angegebenen Bedeutungen haben und
X¹ für Halogen steht, mit Sulfonamiden der allgemeinen Formel (V) 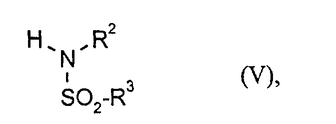 in welcher
- R² und R³: die in Anspruch 1 oder 2 angegebenen Bedeutungen haben,
gegebenenfalls in Gegenwart eines Verdünnungsmittels und gegebenenfalls in Gegenwart eines Säureakzeptors umsetzt.

Die neuen Heterocyclylbenzonitrile der allgemeinen Formel (I) zeichnen sich durch starke herbizide Wirksamkeit aus.

Überraschenderweise zeigen die erfindungsgemäßen Verbindungen der Formel (I) bei guter Verträglichkeit gegenüber Kulturpflanzen, wie z.B. Gerste, erheblich stärkere Wirkung gegen Unkräuter, als die aus dem Stand der Technik bekannten, strukturell ähnlichen Verbindungen 2,5-Difluor-4-(4,5,6,7-tetrahydro-3-methyl-2H-indazol-2-yl)-benzonitril und 4-[5-(t-Butyl)-2-oxo-1,3,4-oxadiazol-3(2H)-yl]-2,5-difluor-benzonitril.

In den Definitionen sind die gesättigten oder ungesättigten Kohlenwasserstoffketten, wie Alkyl, Alkenyl oder Alkinyl jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die Erfindung betrifft vorzugsweise Verbindungen der Formel (I), in welcher
R¹ für Wasserstoff, Fluor oder Chlor steht,
R² für Wasserstoff, für Formyl oder für jeweils gegebenenfalls durch Fluor, Chlor, Cyano, Methoxy, Ethoxy, Methoxycarbonyl oder Ethoxycarbonyl substituiertes Methyl, Ethyl, n- oder i-Propyl, n-, i-, s- oder t-Butyl, Propenyl, Butenyl, Propinyl, Butinyl, Methoxy, Ethoxy, n- oder i-Propoxy, n-, i- oder s-Butoxy, Acetyl, Propionyl, Butyroyl, Methoxycarbonyl, Ethoxycarbonyl, n- oder i-Propoxycarbonyl, Methylsulfonyl, Ethylsulfonyl, n- oder i-Propylsulfonyl, n-, i-, s- oder t-Butylsulfonyl, oder für jeweils gegebenenfalls durch Fluor, Chlor, Brom, Cyano, Methyl, Ethyl, n- oder i-Propyl substituiertes Cyclopropyl, Cyclobutyl, Cyclopentyl, Cyclohexyl, Cyclopropylmethyl, Cyclobutylmethyl, Cyclopentylmethyl, Cyclohexylmethyl, Cyclopropylcarbonyl, Cyclobutylcarbonyl, Cyclopentylcarbonyl, Cyclohexylcarbonyl, Cyclopropylsulfonyl, Cyclobutylsulfonyl, Cyclopentylsulfonyl oder Cyclohexylsulfonyl, oder für Phenylmethyl, Phenylcarbonyl, Phenylmethylcarbonyl, Phenoxycarbonyl, Phenylsulfonyl, Phenylmethylsulfonyl, Thienylsulfonyl, Pyrazolylsulfonyl oder Pyridinylsulfonyl (welche jeweils gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, Difluormethoxy, Trifluormethoxy, Methoxycarbonyl oder Ethoxycarbonyl substituiert sind) steht,
R³ für jeweils gegebenenfalls durch Fluor, Chlor, Cyano, Methoxy oder Ethoxy substituiertes Methyl, Ethyl, n- oder i-Propyl, n-, i-, s- oder t-Butyl, Propenyl, Butenyl, Propinyl, Butinyl, oder für jeweils gegebenenfalls durch Fluor, Chlor, Brom, Cyano, Methyl, Ethyl, n- oder i-Propyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Fluor, Chlor, Brom, Cyano, Nitro, Carboxy, durch Methyl, Ethyl, n- oder i-Propyl, n-, i-, s- oder t-Butyl, Methoxy, Ethoxy, Methylthio, Ethylthio, Methylsulfinyl, Ethylsulfinyl, Methylsulfonyl oder Ethylsulfonyl (welche jeweils gegebenenfalls durch Fluor und/oder Chlor substituiert sind), durch Dimethylaminosulfonyl oder Dimethylaminocarbonyl, durch Methoxycarbonyl oder Ethoxycarbonyl (welche jeweils gegebenenfalls durch Fluor, Chlor, Methoxy oder Ethoxy substituiert sind), durch Phenyl oder Phenoxy substituiertes Phenyl oder Phenylmethyl, oder für jeweils gegebenenfalls durch Fluor, Chlor, Brom, Cyano, Nitro, durch Methyl, Ethyl, n- oder i-Propyl, n-, i-, s- oder t-Butyl, Methoxy, Ethoxy, n- oder i-Propoxy, Methylthio, Ethylthio, Methylsulfinyl, Ethylsulfinyl, Methylsulfonyl, Ethylsulfonyl, Methoxycarbonyl oder Ethoxycarbonyl (welche jeweils gegebenenfalls durch Fluor und/oder Chlor substituiert sind), durch Phenyl oder Phenoxy substituiertes Thienyl, Pyrazolyl, Pyridinyl oder Pyridinylmethyl steht, und
Het für eine der im folgenden aufgeführten (über N gebundenen) heterocyclischen Gruppierungen steht, 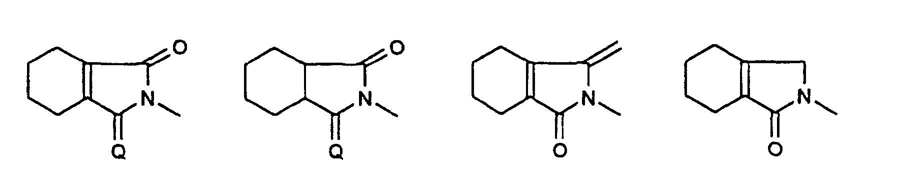 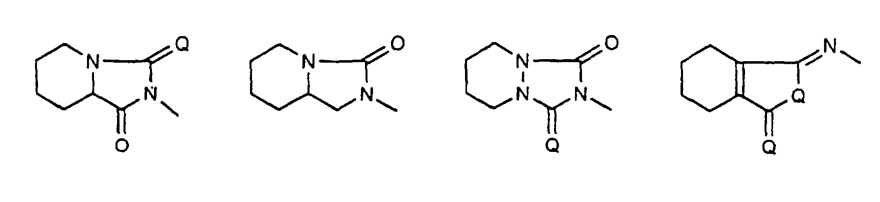 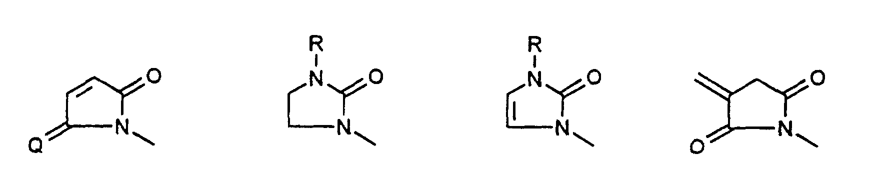 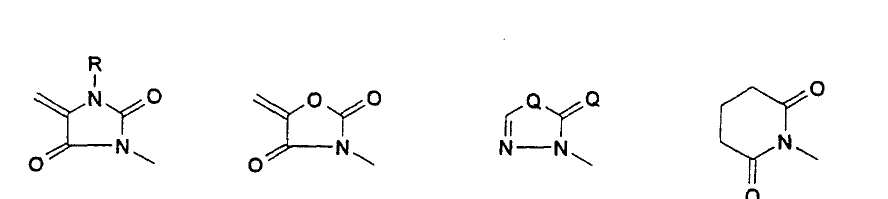 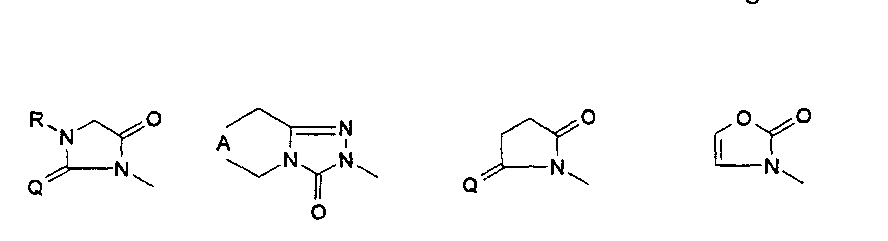 worin jeweils gegebenenfalls

- A: für Methylen, Dimethylen oder Trimethylen steht und
- Q: für Sauerstoff steht und wobei die angegebenen heterocyclischen Gruppierungen jeweils gegebenenfalls einfach oder zweifach substituiert sind durch gleiche oder verschiedene Reste aus der Reihe Hydroxy, Fluor, Chlor, Brom, Cyano, Nitro, Methyl, Ethyl, n- oder i-Propyl, Trifluormethyl, Methoxy, Ethoxy, n- oder i-Propoxy, Difluormethoxy, Trifluormethoxy oder Phenyl und
- R: für einen Rest der Reihe Wasserstoff, Hydroxy, Cyano, Nitro, C₁-C₄-Alkyl, C₁-C₄-Halogenalkyl, C₁-C₄-Alkoxy, C₁-C₄-Halogenalkoxy oder Phenyl steht.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Verwendet man beispielsweise Dimethylmaleinsäureanhydrid und 4-Amino-5-fluor-2-(bis-methylsulfonyl)-amino-benzonitril als Ausgangsstoffe, so kann der Reaktionsablauf beim erfindungsgemäßen Verfahren (a) durch das folgende Formelschema skizziert werden:

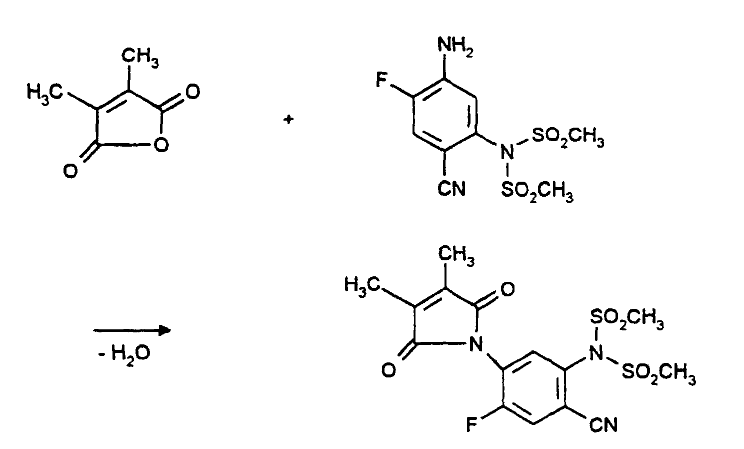

Verwendet man beispielsweise N(4-Cyano-2,5-difluor-phenyl)-3,4,5,6-tetrahydrophthalimid und Methansulfonsäureamid als Ausgangsstoffe, so kann der Reaktionsablauf beim erfindungsgemäßen Verfahren (b) durch das folgende Formelschema skizziert werden:

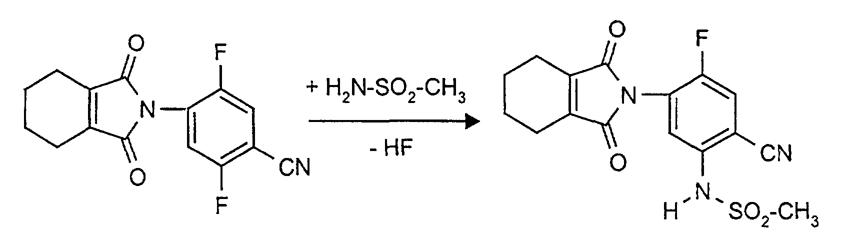

Die beim erfindungsgemäßen Verfahren (a) zur Herstellung der Verbindungen der allgemeinen Formel (I) als Ausgangsstoffe zu verwendenden Anhydride sind durch die Formeln (IIa) bis (IIg) allgemein definiert. In den Formeln (IIa) bis (IIg) hat Q vorzugsweise diejenige Bedeutung, die bereits oben im Zusammenhang mit der Beschreibung der Verbindungen der Formel (I) als bevorzugt für Q angegeben wurde.

Die Ausgangsstoffe der Formeln (IIa) bis (IIg) sind bekannte organische Synthesechemikalien.

Die beim erfindungsgemäßen Verfahren (a) weiter als Ausgangsstoffe zu verwendenden Aminobenzonitrile sind durch die Formel (III) allgemein definiert. In der Formel (III) haben R¹, R² und R³ vorzugsweise diejenigen Bedeutungen, die bereits oben im Zusammenhang mit der Beschreibung der Verbindungen der Formel (I) als bevorzugt für R¹, R² und R³ angegeben wurden.

Die Ausgangsstoffe der Formel (III), in welcher R² nicht für Wasserstoff oder Alkyl steht, sind noch nicht aus der Literatur bekannt; sie sind jedoch Gegenstand einer vorgängigen, nicht vorveröffentlichten Patentanmeldung (vgl. DE-P 4335438 vom 18.10.1993).

Man erhält die Aminobenzonitrile der Formel (III), wenn man entsprechende Halogenverbindungen der allgemeinen Formel (VI) 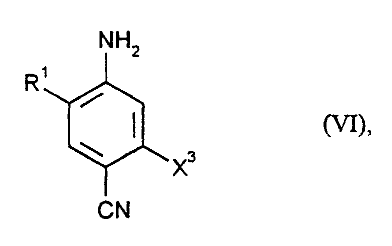 in welcher
- R¹: die oben angegebene Bedeutung hat und
- X³: für Halogen (insbesondere für Fluor oder Chlor) steht,
mit Sulfonamiden der allgemeinen Formel (V) - oben - gegebenenfalls in Gegenwart eines Säureakzeptors, wie z.B. Natriumhydrid, Kaliumcarbonat oder Kalium-t-butylat, und gegebenenfalls in Gegenwart eines Verdünnungsmittels, wie z.B. N-Methyl-pyrrolidon oder Dimethylsulfoxid, bei Temperaturen zwischen 100°C und 200°C umsetzt.

Als Verdünnungsmittel zur Durchführung des erfindungsgemäßen Verfahrens (a) kommen die üblichen organischen Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Carbonsäuren, wie Essigsäure oder Propionsäure; Carbonsäureamide, wie N,N-Dimethylformamid, N,N-Dimethylacetamid, N-Methylformanilid, N-Methyl-pyrrolidon oder Hexamethylphosphorsäuretriamid; Ester wie Essigsäuremethylester oder Essigsäureethylester, Sulfoxide, wie Dimethylsulfoxid oder Sulfone, wie Tetramethylensulfon.

Das erfindungsgemäße Verfahren (a) kann gegebenenfalls in Gegenwart eines Reaktionshilfsmittels durchgeführt werden. Vorzugsweise verwendet man anorganische oder organische Säuren, wie z.B. Essigsäure, Methansulfonsäure, Benzolsulfonsäure oder p-Toluolsulfonsäure, Anhydride, wie z.B. Acetanhydrid, oder Säurechloride, wie z.B. Acetylchlorid als Reaktionshilfsmittel. Es ist auch möglich, andere dehydratisierende Mittel, wie z.B. Dicyclohexylcarbodiimid, oder Acylierungskatalysatoren, wie z.B. 4-Dimethylamino-pyridin als Reaktionshilfsmittel einzusetzen.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 200°C, vorzugsweise bei Temperaturen zwischen 20°C und 150°C.

Das erfindungsgemäße Verfahren (a) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Reaktionshilfsmittels durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Die beim erfindungsgemäßen Verfahren (b) zur Herstellung der Verbindungen der allgemeinen Formel (I) als Ausgangsstoffe zu verwendenden halogenierten Heterocyclylbenzonitrile sind durch die Formel (IV) allgemein definiert. In der Formel (IV) haben Het und R¹ vorzugsweise bzw. insbesondere diejenigen Bedeutungen, die bereits oben im Zusammenhang mit der Beschreibung der Verbindungen der Formel
(I) als bevorzugt bzw. als insbesondere bevorzugt für Het und R¹ angegeben wurden; X¹ steht vorzugsweise für Fluor, Chlor oder Brom, insbesondere für Fluor oder Chlor.

Die Ausgangsstoffe der Formel (IV) sind bekannt und/oder können nach bekannten Verfahren hergestellt werden (vgl. EP-A 364797; Herstellungsbeispiele).

Die beim erfindungsgemäßen Verfahren (b) weiter als Ausgangsstoffe zu verwendenden Sulfonamide sind durch die Formel (V) allgemein definiert. In der Formel (V) haben R² und R³ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R² und R³ angegeben wurden.

Die Ausgangsstoffe der Formel (V) sind bekannte organische Synthesechemikalien.

Als Verdünnungsmittel zur Durchführung des erfindungsgemäßen Verfahrens (b) kommen die üblichen organischen Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofüran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wieN,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether.

Das erfindungsgemäße Verfahren (b) wird vorzugsweise in Gegenwart eines geeigneten Säureakzeptors durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie basische organische Stickstoffverbindungen, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen 0°C und 200°C, vorzugsweise bei Temperaturen zwischen 10°C und 150°C.

Das erfindungsgemäße Verfahren (b) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindemia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen und dikotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

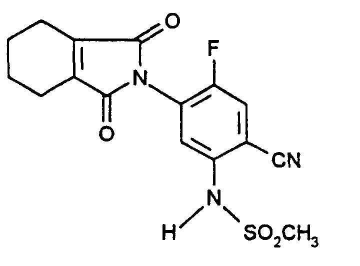

### (Verfahren (a))

Eine Mischung aus 0,76 g (5 mMol) 3,4,5,6-Tetrahydrophthalsäureanhydrid, 1,15 g (5 mMol) 4-Amino-5-fluor-2-methylsulfonylamino-benzonitril und 10 ml Essigsäure wird mit einer Spatelspitze 4-Dimethylamino-pyridin 30 Stunden unter Rückfluß (ca. 120°C) erhitzt und anschließend eingeengt. Der Rückstand wird mit einer Mischung aus Wasser, Diethylether und Essigsäureethylester digeriert und das kristallin anfallende Produkt durch Absaugen isoliert.

Man erhält 0,60 g (33% der Theorie) N-(4-Cyano-2-fluor-5-methylsulfonylaminophenyl)-3,4,5,6-tetrahydrophthalimid vom Schmelzpunkt 113°C.

### Beispiel 2

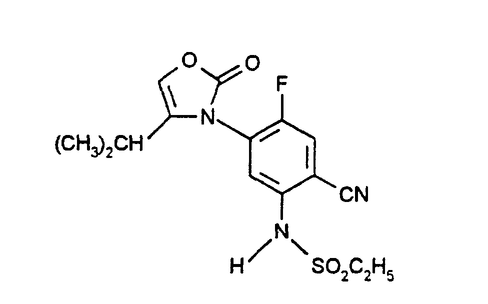

### (Verfahren (b))

Eine Mischung aus 0,80 g (3,0 mMol) 3-(4-Cyano-2,5-difluor-phenyl)-4-isopropyloxazolin-2-on, 0,36 g (3,3 mMol) Ethansulfonamid, 0,46 g (3,3 mMol) Kaliumcarbonat und 50 ml Dimethylsulfoxid wird 3 Stunden bei 120°C gerührt, anschließend auf Eiswasser gegossen, mit konzentrierter Salzsäure angesäuert und mit Methylenchlorid geschüttelt. Die organische Phase wird abgetrennt, mit Natriumsulfat getrocknet und filtriert. Vom Filtrat wird das Lösungsmittel im Wasserstrahlvakuum sorgfältig abdestilliert.

Man erhält 0,60 g (57 % der Theorie) 3-(4-Cyano-2-fluor-5-ethylsulfonylamino-phenyl)-4-isopropyl-oxazolin-2-on als öligen Rückstand.

Analog zu den Beispielen 1 und 2 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

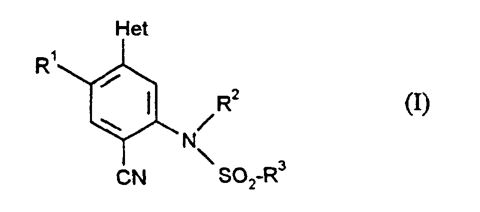

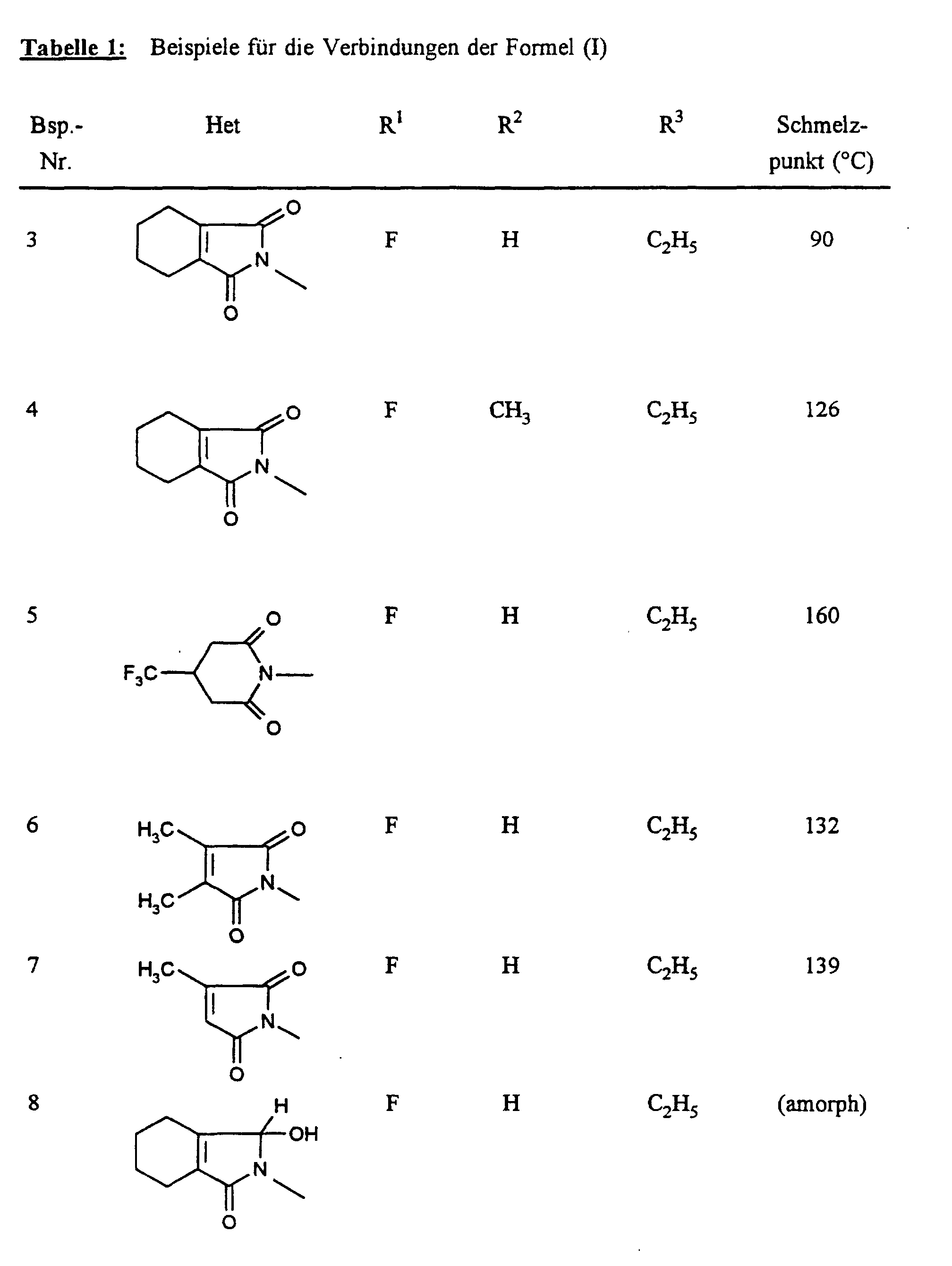

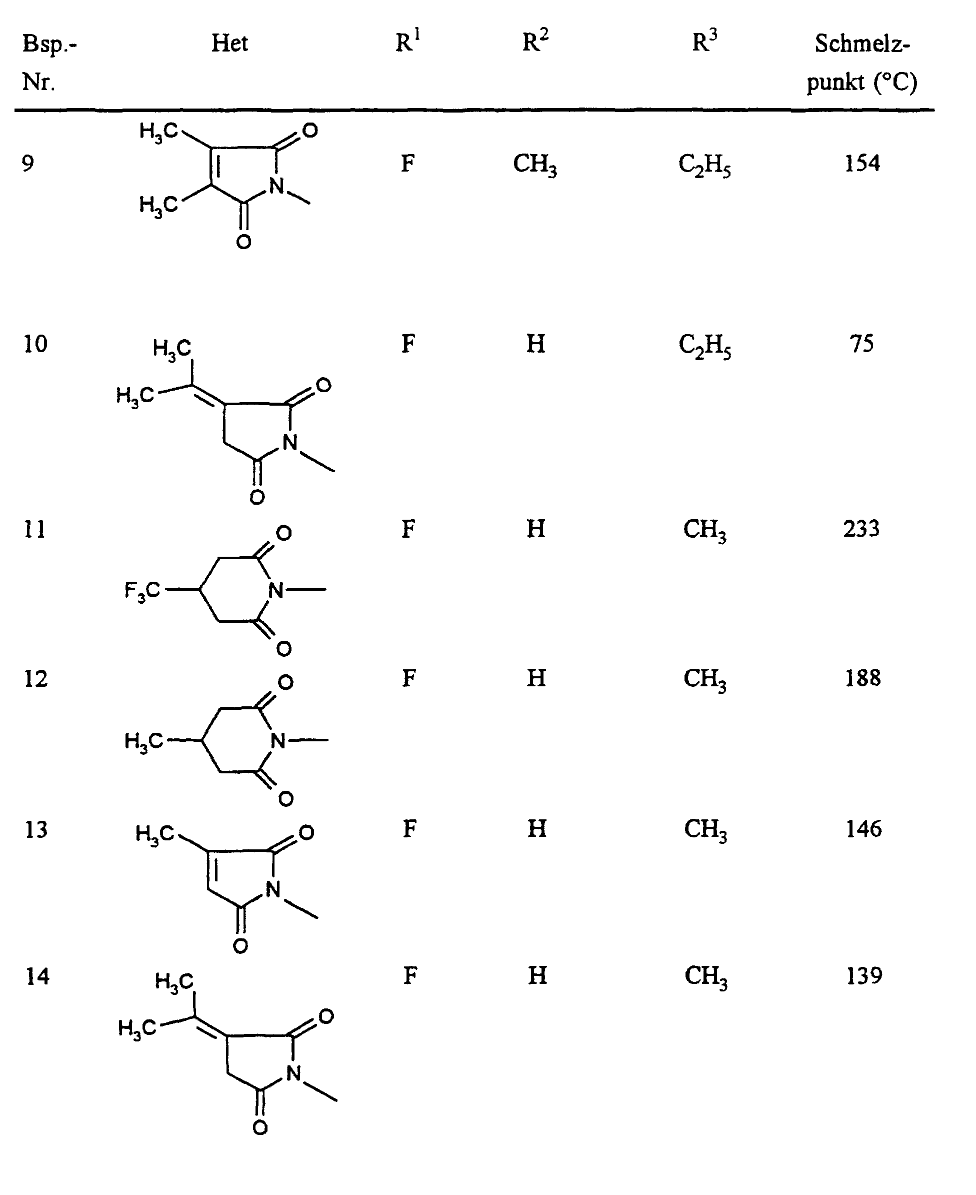

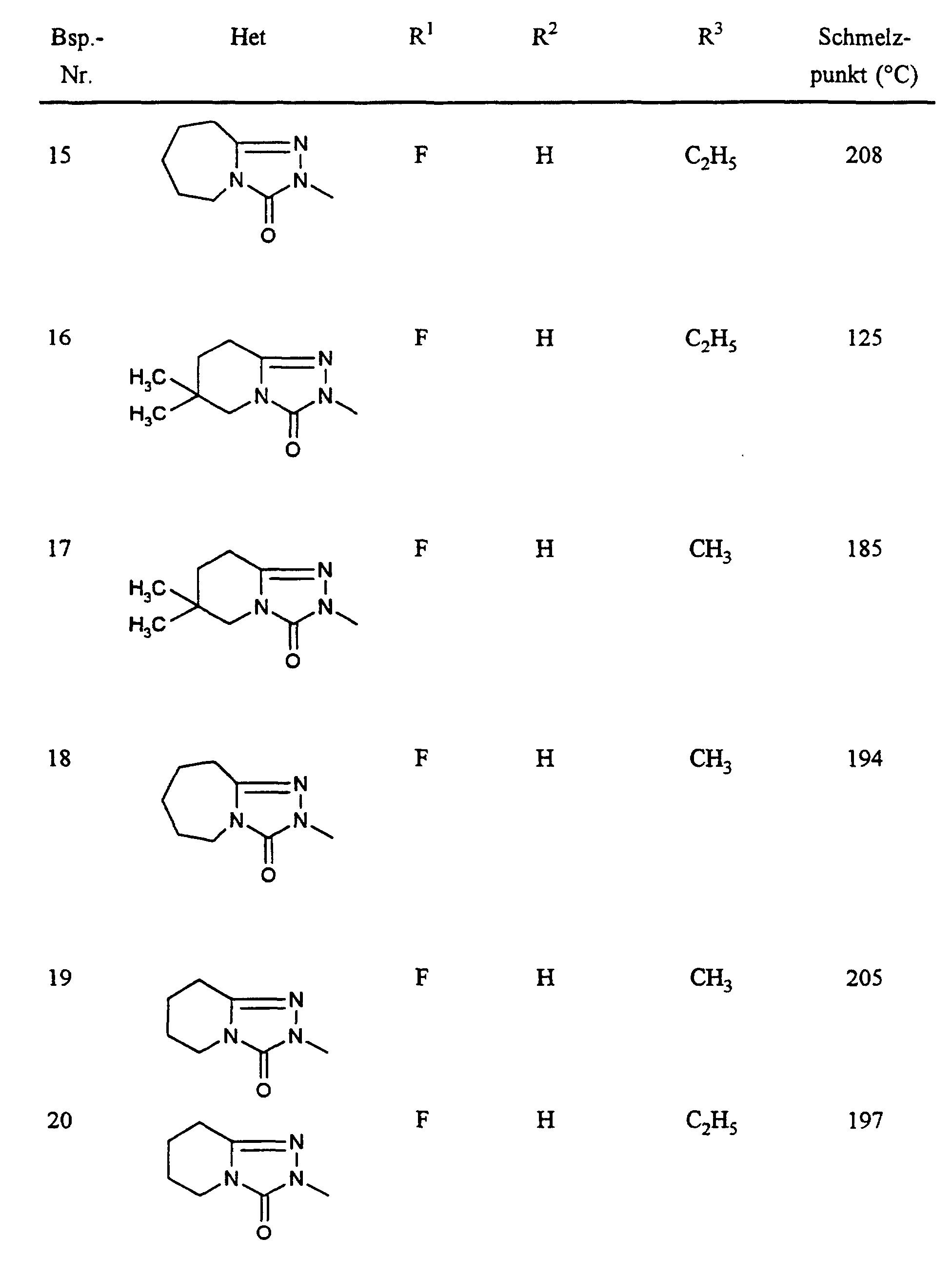

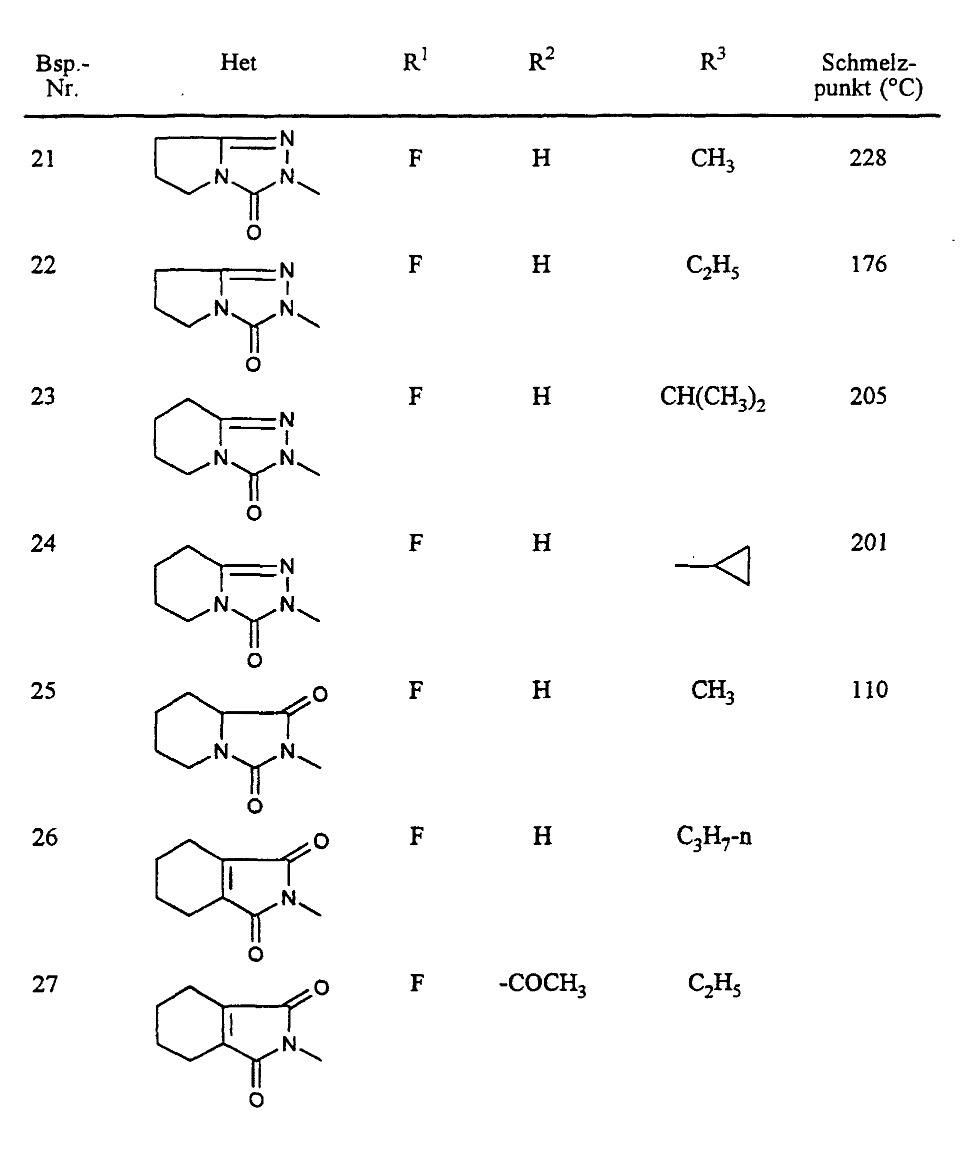

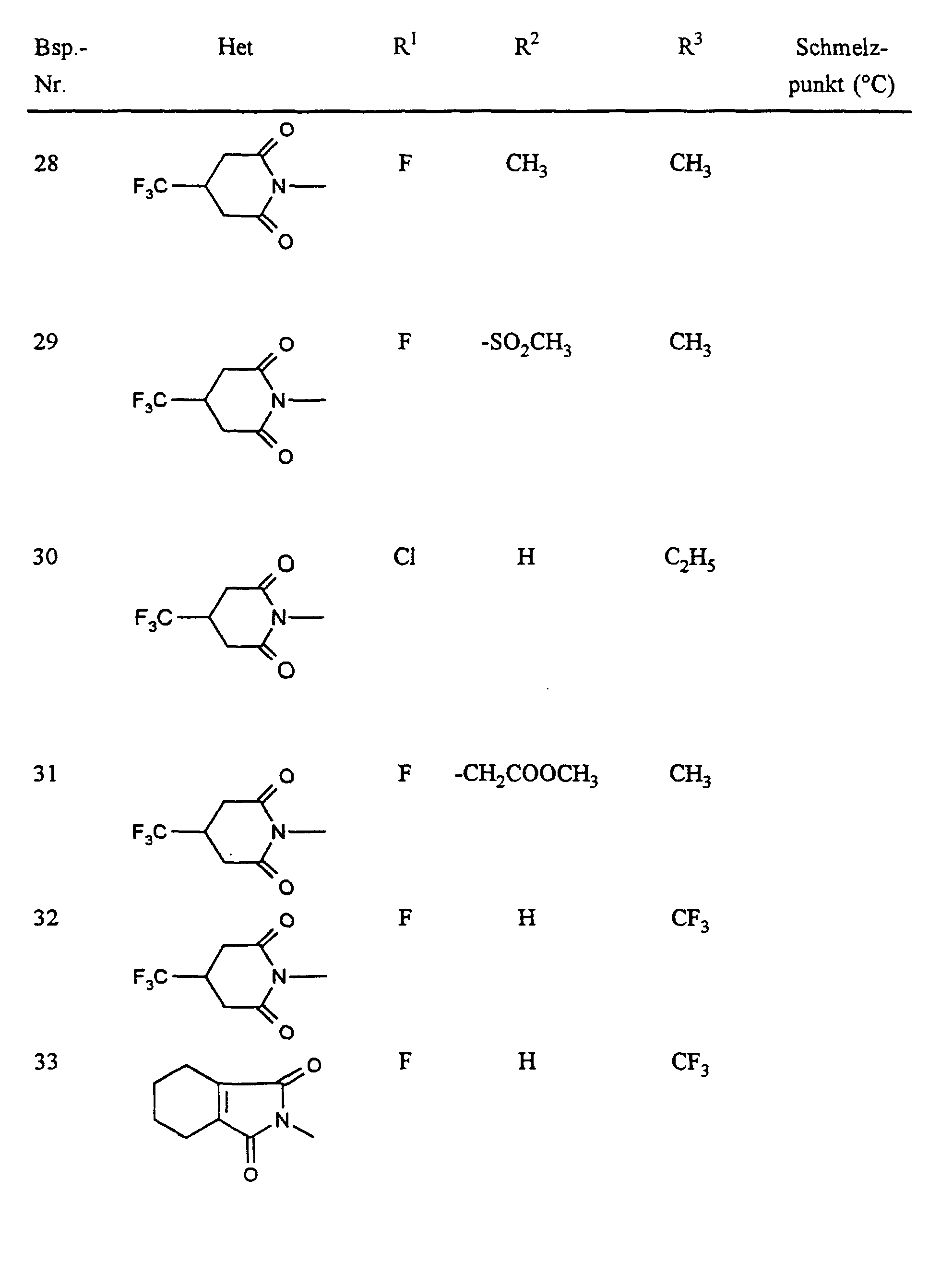

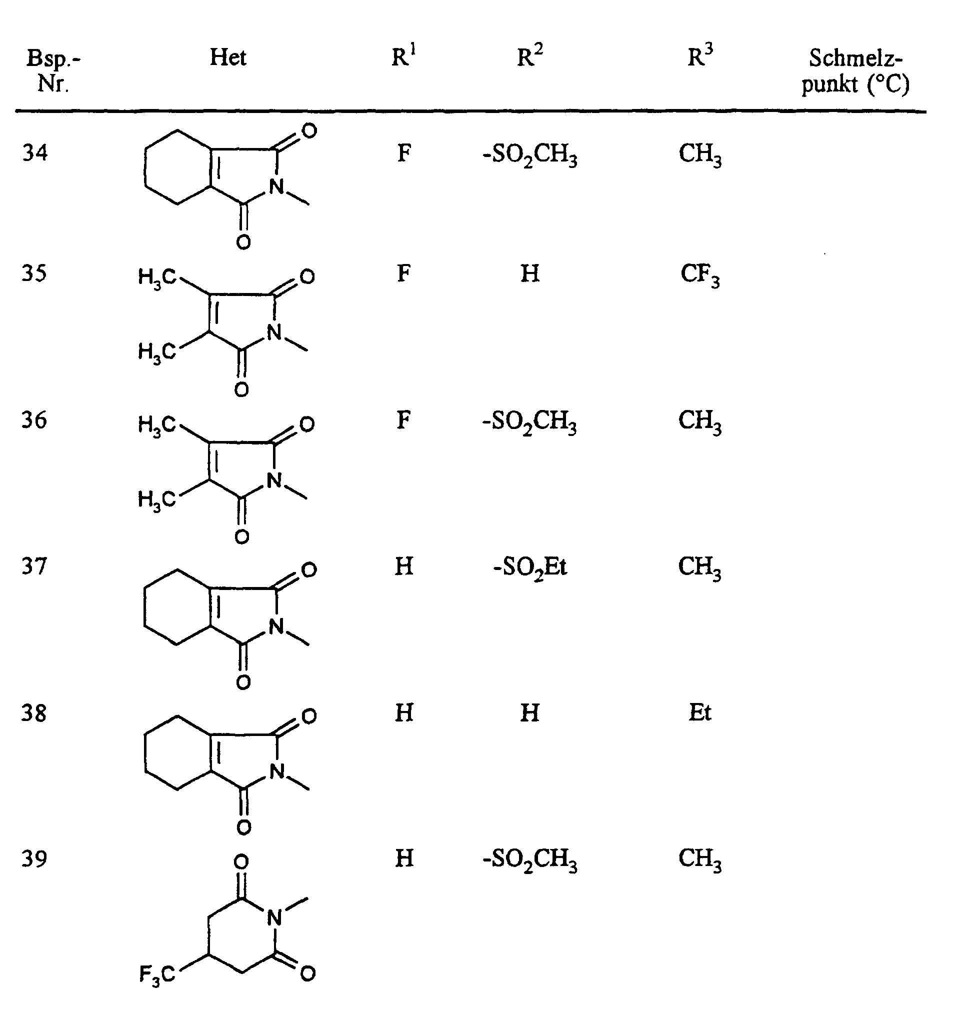

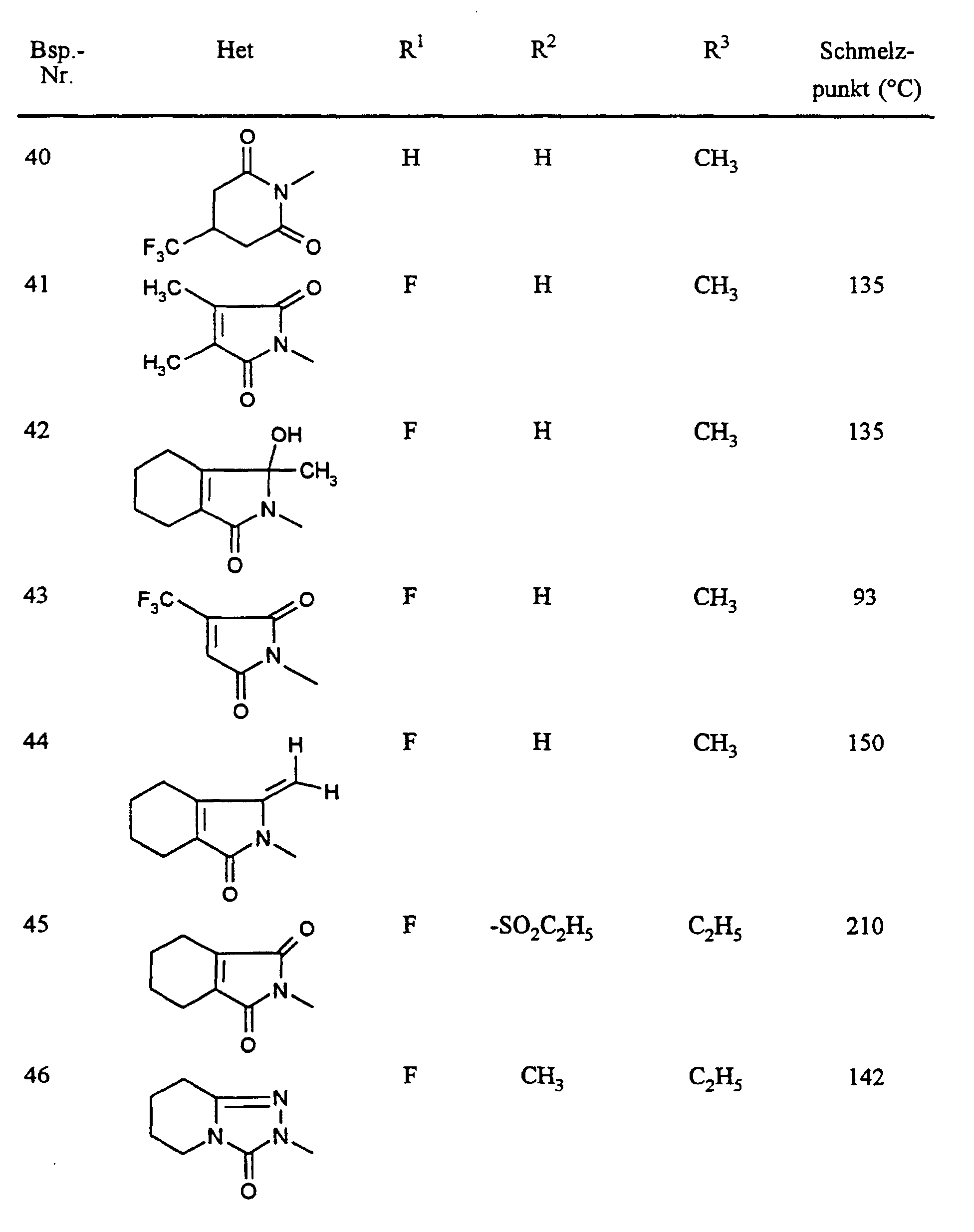

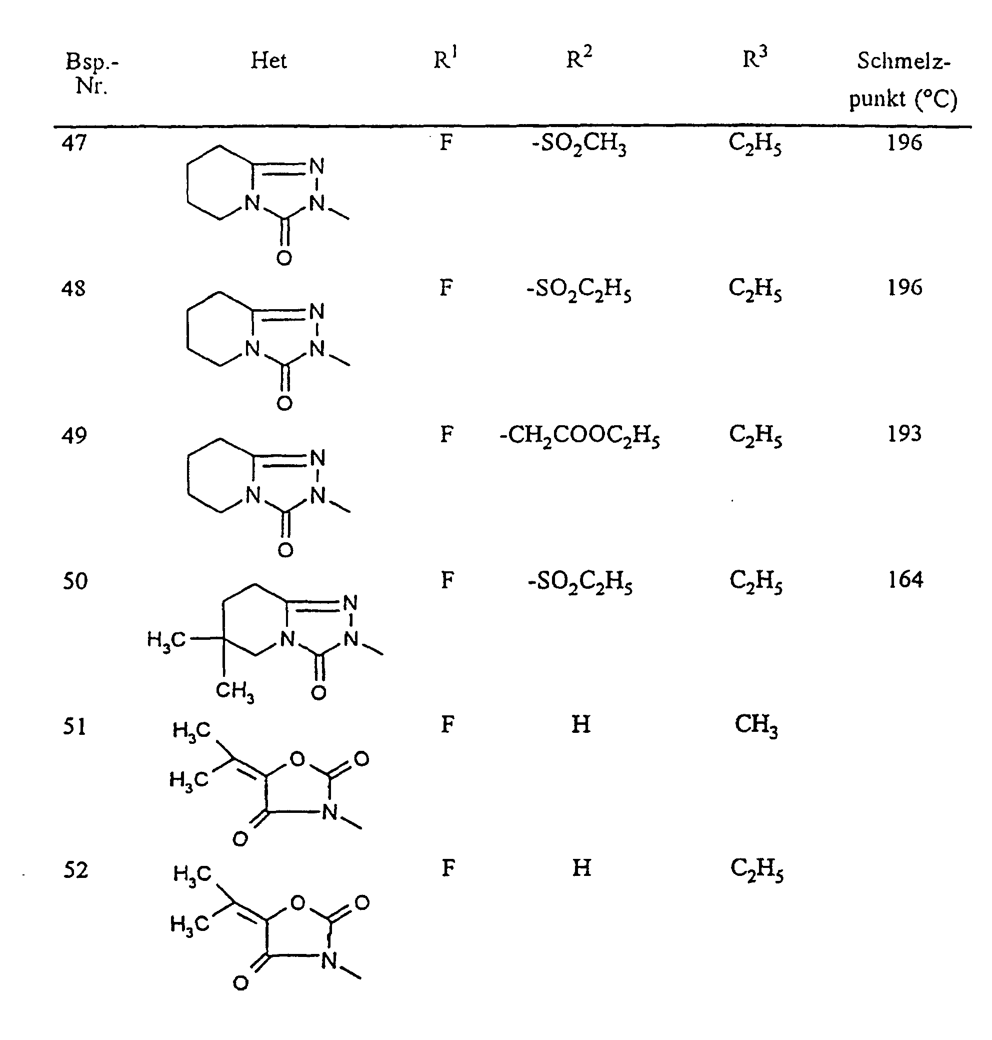

### Ausgangsstoffe der Formel (III):

### Beispiel (III-1)

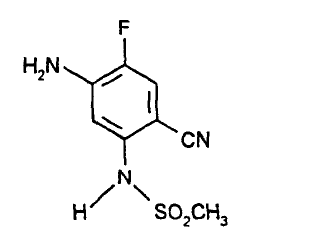

Eine Mischung aus 92,4 g (0,6 Mol) 4-Cyano-2,5-difluor-anilin, 60 g (0,60 Mol) Methansulfonamid, 166 g Kaliumcarbonat und 80 ml N-Methyl-pyrrolidonwird 10 Stunden auf 180°C erhitzt. Nach dem Erkalten wird die Mischung in 5 Liter Wasser eingerührt und die erhaltene Lösung zweimal mit je 400 ml Essigsäureethylester gewaschen. Die wäßrige Phase wird dann mit 300 ml Essigsäureethylester überschichtet und mit 10%iger Salzsäure angesäuert. Das kristallin anfallende Produkt wird dann durch Absaugen isoliert.

Man erhält 70 g (51 % der Theorie) N-(5-Amino-2-cyano-4-fluor-phenyl)-methansulfonamid vom Schmelzpunkt 238°C.

### Ausgangsstoffe der Formel (IV):

### Beispiel (IV-1)

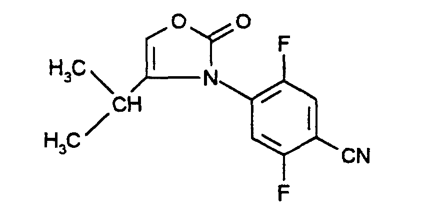

Eine Mischung aus 2,0 g (16 mMol) 4-Isopropyl-oxazolin-2-on, 2,5 g (16 mMol) 2,4,5-Trifluor-benzonitril, 2,8 g (20 mMol) Kaliumcarbonat und 50 ml Dimethylsulfoxid wird 15 Stunden bei 60°C gerührt, anschließend mit Wasser auf etwa das dreifache Volumen verdünnt und mit konzentrierter Salzsäure angesäuert. Die organische Phase wird abgetrennt, mit Methylenchlorid verdünnt, mit Natriumsulfat getrocknet und filtriert. Das Filtrat wird eingeengt und der Rückstand aus Isopropanol umkristallisiert.

Man erhält 0,80 g (19% der Theorie) 3-(4-Cyano-2,5-difluor-phenyl)-4-isopropyl-oxazolin-2-on vom Schmelzpunkt 83°C.

Analog Beispiel (IV-1) können beispielsweise auch die in der nachstehenden Tabelle 2 aufgeführten Verbindungen der Formel (IV) hergestellt werden.

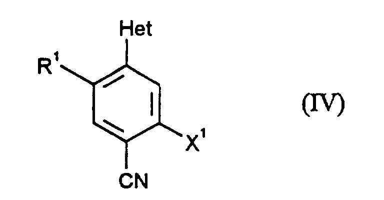

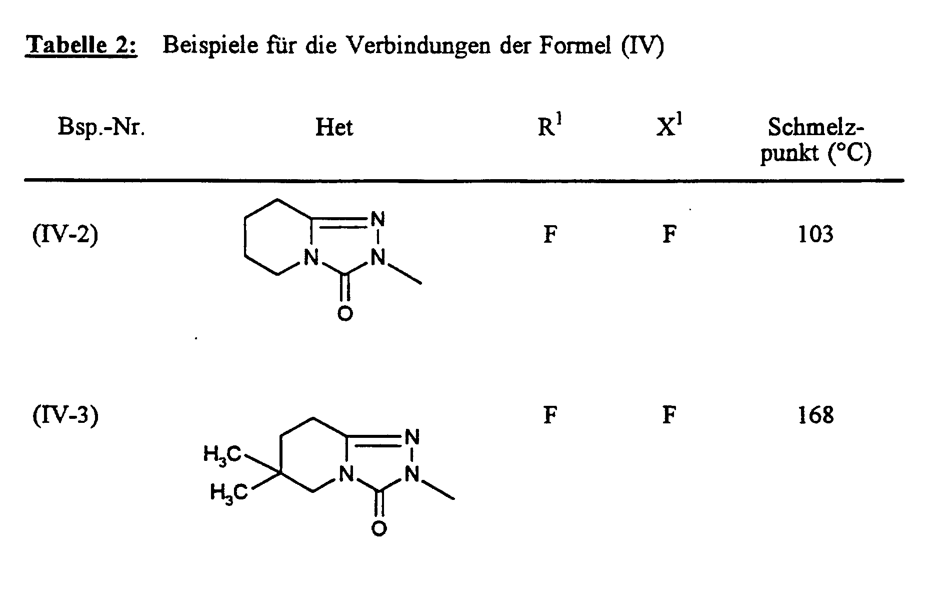

### Anwendungsbeispiele:

In den nachfolgenden Anwendungsbeispielen werden die folgenden Verbindungen als Vergleichssubstanz herangezogen:

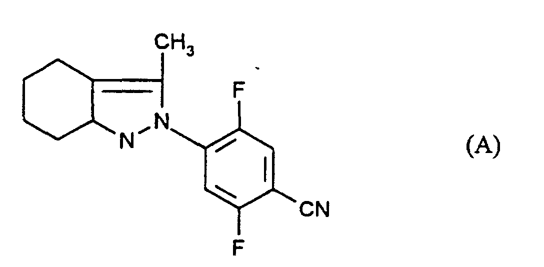

2,5-Difluor-4-(4,5,6,7-tetrahydro-3-methyl-2H-indazol-2-yl)-benzonitril (bekannt aus EP-A 370332, Beispiel 2).

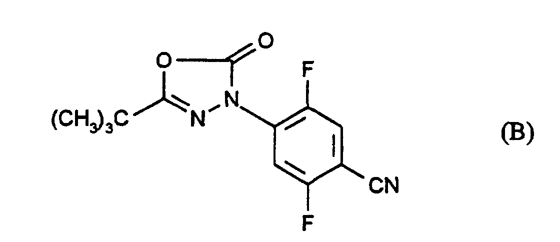

4-[5-(t-Butyl)-2-oxo-1,3,4-oxadiazol-3(2H)-yl]-2,5-difluor-benzonitril (bekannt aus EP-A 370332, Beispiel 7).

### Beispiel A

Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach 24 Stunden wird der Boden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen beispielsweise die Verbindungen gemäß den Herstellungsbeispielen 3 und 5 bei sehr guter Verträglichkeitund und Aufwandmengen von 125 g/ha gegenüber Kulturpflanzen, wie z.B. Gerste (0 %), starke Wirkung gegen Unkräuter wie Abutilon (100 %), Amaranthus (100 %), Chenopodium (90-100 %), Datura (80-100 %), Galinsoga (95-100 %), Portulaca (100 %) und Solanum (80-100 %).

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:
0% = keine Wirkung (wie unbehandelte Kontrolle)
100 % = totale Vernichtung

In diesem Test zeigen beispielsweise die Verbindungen gemäß den Herstellungsbeispielen 1, 3 und 5 bei Aufwandmengen von 30 g/ha und bei sehr guter Verträglichkeit gegenüber Kulturpflanzen, wie z.B. Gerste, sehr starke Wirkung gegen Unkräuter wie Abutilon (100 %), Amaranthus (100 %), Chenopodium (95 %), Galium (90-95 %) und Veronica (100 %).

## Patentansprüche

1. Heterocyclylbenzonirile der allgemeinen Formel (I), 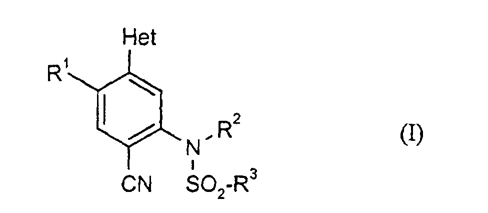 in welcher
R¹ für Wasserstoff oder Halogen steht,
R² für Wasserstoff, für Formyl oder für jeweils gegebenenfalls durch Halogen, Cyano, C₁-C₄-Alkoxy oder C₁-C₄-Alkoxy-carbonyl substituiertes Alkyl, Alkenyl, Alkinyl, Alkoxy, Alkylcarbonyl, Alkoxycarbonyl oder Alkylsulfonyl mit jeweils bis zu 6 Kohlenstoffatomen, für jeweils gegebenenfalls durch Halogen, Cyano oder C₁-C₄-Alkyl substituiertes Cycloalkyl, Cycloalkylalkyl, Cycloalkylcarbonyl oder Cycloalkylsulfonyl mit 3 bis 6 Kohlenstoffatomen im Cycloalkylteil und gegebenenfalls 1 bis 4 Kohlenstoffatomen im Alkylteil, oder für Phenylmethyl, Phenylcarbonyl, Naphthylcarbonyl, Phenylmethylcarbonyl, Phenoxycarbonyl, Phenylsulfonyl, Naphthylsulfonyl, Phenylmethylsulfonyl, Thienylsulfonyl, Pyrazolylsulfonyl, Pyridinylsulfonyl oder Pyridinylmethylsulfonyl (welche jeweils gegebenenfalls durch Halogen, Cyano, C₁-C₄-Alkyl, C₁-C₄-Halogenalkyl, C₁-C₄-Alkoxy, C₁-C₄-Halogenalkoxy oder C₁-C₄-Alkoxycarbonyl substituiert sind) steht,
R³ für jeweils gegebenenfalls durch Halogen, Cyano oder C₁-C₄-Alkoxy substituiertes Alkyl, Alkenyl oder Alkinyl mit jeweils bis zu 10 Kohlenstoffatomen steht, oder für jeweils gegebenenfalls durch Halogen, Cyano oder C₁-C₄-Alkyl substituiertes Cycloalkyl oder Cycloalkylalkyl mit 3 bis 8 Kohlenstoffatomen im Cycloalkylteil und gegebenenfalls 1 bis 4 Kohlenstoffatomen im Alkylteil steht, oder für jeweils gegebenenfalls durch Halogen, Cyano, Nitro, Carboxy, Carbamoyl, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Fluor und/oder Chlor substituiert sind), durch Dimethylaminosulfonyl, Diethylaminosulfonyl, Dimethylaminocarbonyl oder Diethylaminocarbonyl, durch C₁-C₄-Alkoxy-carbonyl (welches gegebenenfalls durch Halogen, Methoxy oder Ethoxy substituiert ist), durch Phenyl, Phenyloxy oder Phenylthio (welche jeweils gegebenenfalls durch Halogen, Cyano, Methyl, Methoxy, Trifluormethyl und/oder Trifluormethoxy substituiert sind) substituiertes Aryl oder Arylalkyl mit 6 oder 10 Kohlenstoffatomen im Arylteil und 1 bis 4 Kohlenstoffatomen im Alkylteil, oder für jeweils gegebenenfalls durch Halogen, Cyano, Nitro, Carboxy, Carbamoyl, durch C₁-C₄-Alkyl, C₁-C₄-Alkoxy, C₁-C₄-Alkylthio, C₁-C₄-Alkylsulfinyl, C₁-C₄-Alkylsulfonyl oder C₁-C₄-Alkoxycarbonyl (welche jeweils gegebenenfalls durch Halogen substituiert sind), durch Phenyl, Phenoxy oder Phenylthio (welche jeweils gegebenenfalls durch Halogen, Cyano, C₁-C₄-Alkyl, C₁-C₄-Halogenalkyl, C₁-C₄-Alkoxy und/oder C₁-C₄-Halogenalkoxy substituiert sind) substituiertes Heterocyclyl oder Heterocyclylalkyl mit 2 bis 6 Kohlenstoffatomen und 1 bis 4 Stickstoffatomen und/oder 1 bis 2 Sauerstoff- oder Schwefelatomen im gesättigten oder ungesättigten Heterocyclylteil und gegebenenfalls 1 bis 4 Kohlenstoffatomen im Alkylteil steht, und
Het für eine der im folgenden aufgeführten (über N gebundenen) heterocyclischen Gruppierungen steht, 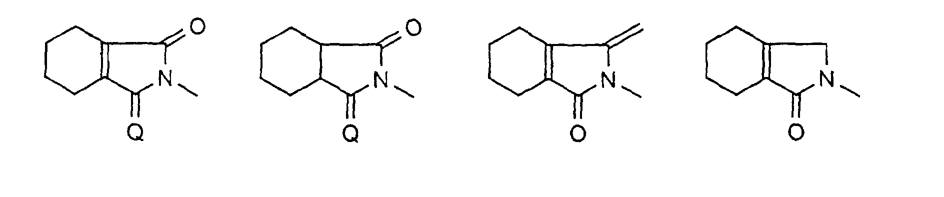 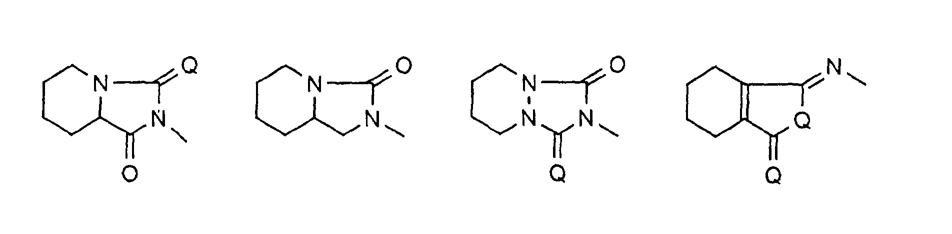 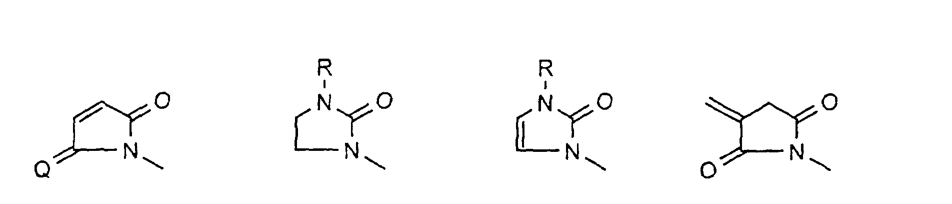 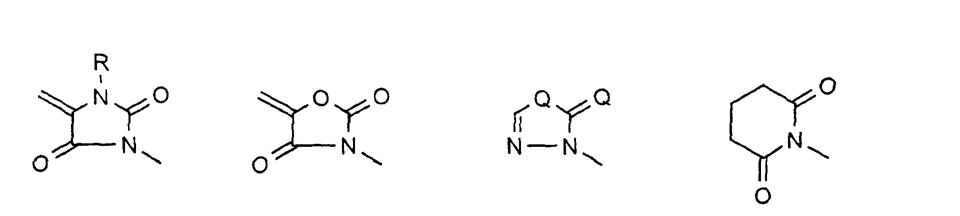 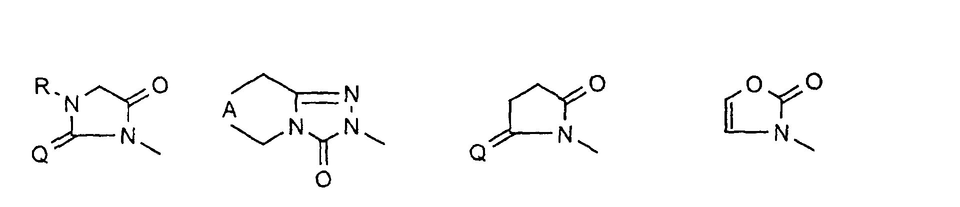 worin jeweils gegebenenfalls
A für gegebenenfalls durch SO₂ unterbrochenes C₁-C₃-Alkandiyl steht und
Q für Sauerstoff oder Schwefel steht und wobei die angegebenen heterocyclischen Gruppierungen jeweils gegebenenfalls einfach bis dreifach substituiert sind durch gleiche oder verschiedene Reste aus der Reihe Hydroxy, Halogen, Cyano, Nitro, C₁-C₄-Alkyl, C₁-C₄-Halogenalkyl, C₁-C₄-Alkoxy, C₁-C₄-Halogenalkoxy oder Phenyl und
R für einen Rest aus der Reihe Wasserstoff, Hydroxy, Cyano, Nitro, C₁C₄-Alkyl, C₁-C₄-Halogenalkyl, C₁-C₄-Alkoxy, C₁-C₄-Halogenalkoxy oder Phenyl steht.

2. Heterocyclylbenzonitrile gemäß Anspruch 1, **dadurch gekennzeichnet, daß**
R¹ für Wasserstoff, Fluor oder Chlor steht,
R² für Wasserstoff, für Formyl oder für jeweils gegebenenfalls durch Fluor, Chlor, Cyano, Methoxy, Ethoxy, Methoxycarbonyl oder Ethoxycarbonyl substituiertes Methyl, Ethyl, n- oder i-Propyl, n-, i-, s- oder t-Butyl, Propenyl, Butenyl, Propinyl, Butinyl, Methoxy, Ethoxy, n- oder i-Propoxy, n-, i- oder s-Butoxy, Acetyl, Propionyl, Butyroyl, Methoxycarbonyl, Ethoxycarbonyl, n- oder i-Propoxycarbonyl, Methylsulfonyl, Ethylsulfonyl, n- oder i-Propylsulfonyl, n-, i-, s- oder t-Butylsulfonyl, oder für jeweils gegebenenfalls durch Fluor, Chlor, Brom, Cyano, Methyl, Ethyl, n- oder i-Propyl substituiertes Cyclopropyl, Cyclobutyl, Cyclopentyl, Cyclohexyl, Cyclopropylmethyl, Cyclobutylmethyl, Cyclopentylmethyl, Cyclohexylmethyl, Cyclopropylcarbonyl, Cyclobutylcarbonyl, Cyclopentylcarbonyl, Cyclohexylcarbonyl, Cyclopropylsulfonyl, Cyclobutylsulfonyl, Cyclopentylsulfonyl oder Cyclohexylsulfonyl, oder für Phenylmethyl, Phenylcarbonyl, Phenylmethylcarbonyl, Phenoxycarbonyl, Phenylsulfonyl, Phenylmethylsulfonyl, Thienylsulfonyl, Pyrazolylsulfonyl oder Pyridinylsulfonyl (welche jeweils gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, Difluormethoxy, Trifluormethoxy, Methoxycarbonyl oder Ethoxycarbonyl substituiert sind) steht,
R³ für jeweils gegebenenfalls durch Fluor, Chlor, Cyano, Methoxy oder Ethoxy substituiertes Methyl, Ethyl, n- oder i-Propyl, n-, i-, s- oder t-Butyl, Propenyl, Butenyl, Propinyl, Butinyl, oder für jeweils gegebenenfalls durch Fluor, Chlor, Brom, Cyano, Methyl, Ethyl, n- oder i-Propyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Fluor, Chlor, Brom, Cyano, Nitro, Carboxy, durch Methyl, Ethyl, n- oder i-Propyl, n-, i-, s- oder t-Butyl, Methoxy, Ethoxy, Methylthio, Ethylthio, Methylsulfinyl, Ethylsulfinyl, Methylsulfonyl oder Ethylsulfonyl (welche jeweils gegebenenfalls durch Fluor und/oder Chlor substituiert sind), durch Dimethylaminosulfonyl oder Dimethylaminocarbonyl, durch Methoxycarbonyl oder Ethoxycarbonyl (welche jeweils gegebenenfalls durch Fluor, Chlor, Methoxy oder Ethoxy substituiert sind), durch Phenyl oder Phenoxy substituiertes Phenyl oder Phenylmethyl, oder für jeweils gegebenenfalls durch Fluor, Chlor, Brom, Cyano, Nitro, durch Methyl, Ethyl, n- oder i-Propyl, n-, i-, s- oder t-Butyl, Methoxy, Ethoxy, n- oder i-Propoxy, Methylthio, Ethylthio, Methylsulfinyl, Ethylsulfinyl, Methylsulfonyl, Ethylsulfonyl, Methoxycarbonyl oder Ethoxycarbonyl (welche jeweils gegebenenfalls durch Fluor und/oder Chlor substituiert sind), durch Phenyl oder Phenoxy substituiertes Thienyl, Pyrazolyl, Pyridinyl oder Pyridinylmethyl steht, und
A für Methylen, Dimethylen oder Trimethylen steht,
Q für Sauerstoff steht und wobei die angegebenen heterocyclischen Gruppierungen jeweils gegebenenfalls einfach oder zweifach substituiert sind durch gleiche oder verschiedene Reste aus der Reihe Hydroxy, Fluor, Chlor, Brom, Cyano, Nitro, Methyl, Ethyl, n- oder i-Propyl, Trifluormethyl, Methoxy, Ethoxy, n- oder i-Propoxy, Difluormethoxy, Trifluormethoxy oder Phenyl und
R für einen Rest der Reihe Wasserstoff, Hydroxy, Cyano, Nitro, C₁-C₄-Alkyl, C₁-C₄-Halogenalkyl, C₁-C₄-Alkoxy, C₁-C₄-Halogenalkoxy oder Phenyl steht.

3. Verfahren zur Herstellung von Heterocyclylbenzonitrilen gemäß Anspruch 1 oder 2, **dadurch gekennzeichnet, daß** man
(a) Anhydride der allgemeinen Formeln (IIa) bis (IIg), 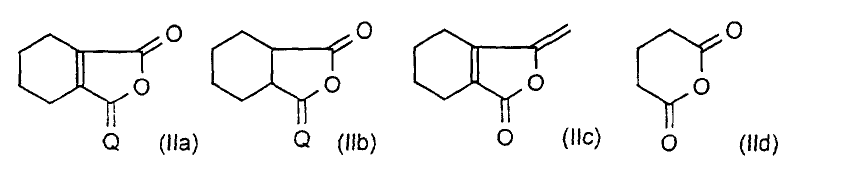 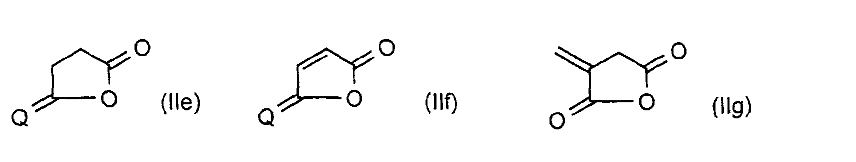 in welcher
Q die in Anspruch 1 oder 2 angegebene Bedeutung hat,
mit Aminobenzonitrilen der allgemeinen Formel (III) 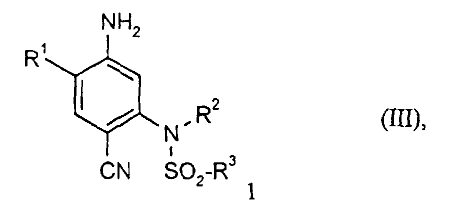 in welcher
R¹, R² und R³ die in Anspruch 1 oder 2 angegebenen Bedeutungen haben,
gegebenenfalls in Gegenwart eines Verdünnungsmittels und gegebenenfalls in Gegenwart eines Reaktionshilfsmittels umsetzt,
oder **daß** man
(b) halogenierte Heterocyclylbenzonitrile der allgemeinen Formel (IV) 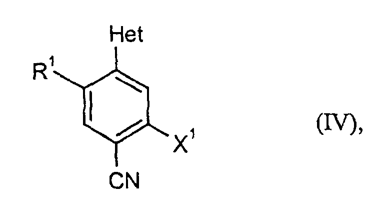 in welcher
Het und R¹ die in Anspruch 1 oder 2 angegebenen Bedeutungen haben und
X¹ für Halogen steht,
mit Sulfonamiden der allgemeinen Formel (V) 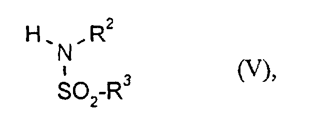 in welcher
R² und R³ die in Anspruch 1 oder 2 angegebenen Bedeutungen haben,
gegebenenfalls in Gegenwart eines Verdünnungsmittels und gegebenenfalls in Gegenwart eines Säureakzeptors umsetzt.

4. Verfahren zur Bekämpfung von unerwünschten Pflanzen, **dadurch gekennzeichnet, daß** man Heterocyclylbenzonitrile gemäß Anspruch 1 oder 2 auf unerwünschte Pflanzen und/oder ihren Lebensraum einwirken läßt.

5. Verwendung von Heterocyclylbenzonitrilen gemäß Anspruch 1 oder 2 zur Bekämpfung von unerwünschten Pflanzen.

6. Verfahren zur Herstellung von herbiziden Mitteln, **dadurch gekennzeichnet, daß** man Heterocyclylbenzonitrile gemäß Anspruch 1 oder 2 mit Streckmitteln und/oder oberflächenaktiven Substanzen vermischt.

7. Herbizide Mittel, **gekennzeichnet durch** einen Gehalt an mindestens einem Heterocyclylbenzonitril gemäß Anspruch 1 oder 2.

8. Aminobenzonitrile der allgemeinen Formel (III) 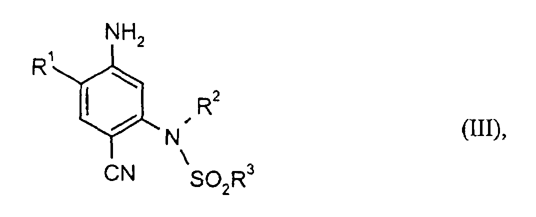 in welcher
R¹ für Wasserstoff oder Halogen steht,
R² für Formyl oder für jeweils gegebenenfalls durch Halogen, Cyano, C₁-C₄-Alkoxy oder C₁-C₄-Alkoxy-carbonyl substituiertes Alkenyl, Alkinyl, Alkoxy, Alkylcarbonyl, Alkoxycarbonyl oder Alkylsulfonyl mit jeweils bis zu 6 Kohlenstoffatomen, für jeweils gegebenenfalls durch Halogen, Cyano oder C₁-C₄-Alkyl substituiertes Cycloalkyl, Cycloalkylalkyl, Cycloalkylcarbonyl oder Cycloalkylsulfonyl mit 3 bis 6 Kohlenstoffatomen im Cycloalkylteil und gegebenenfalls 1 bis 4 Kohlenstoffatomen im Alkylteil, oder für Phenylmethyl, Phenylcarbonyl, Naphthylcarbonyl, Phenylmethylcarbonyl, Phenoxycarbonyl, Phenylsulfonyl, Naphthylsulfonyl, Phenylmethylsulfonyl, Thienylsulfonyl, Pyrazolylsulfonyl, Pyridinylsulfonyl oder Pyridinylmethylsulfonyl (welche jeweils gegebenenfalls durch Halogen, Cyano, C₁-C₄-Alkyl, C₁-C₄-Halogenalkyl, C₁-C₄-Alkoxy, C₁-C₄-Halogenalkoxy oder C₁-C₄-Alkoxycarbonyl substituiert sind) steht, und
R³ für jeweils gegebenenfalls durch Halogen, Cyano oder C₁-C₄-Alkoxy substituiertes Alkyl, Alkenyl oder Alkinyl mit jeweils bis zu 10 Kohlenstoffatomen steht, oder für jeweils gegebenenfalls durch Halogen, Cyano oder C₁-C₄-Alkyl substituiertes Cycloalkyl oder Cycloalkylalkyl mit 3 bis 8 Kohlenstoffatomen im Cycloalkylteil und gegebenenfalls 1 bis 4 Kohlenstoffatomen im Alkylteil steht, oder für jeweils gegebenenfalls durch Halogen, Cyano, Nitro, Carboxy, Carbamoyl, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Fluor und/oder Chlor substituiert sind), durch Dimethylaminosulfonyl, Diethylaminosulfonyl, Dimethylaminocarbonyl oder Diethylaminocarbonyl, durch C₁-C₄-Alkoxy-carbonyl (welches gegebenenfalls durch Halogen, Methoxy oder Ethoxy substituiert ist), durch Phenyl, Phenyloxy oder Phenylthio (welche jeweils gegebenenfalls durch Halogen, Cyano, Methyl, Methoxy, Trifluormethyl und/oder Trifluormethoxy substituiert sind) substituiertes Aryl oder Arylalkyl mit 6 oder 10 Kohlenstoffatomen im Arylteil und 1 bis 4 Kohlenstoffatomen im Alkylteil, oder für jeweils gegebenenfalls durch Halogen, Cyano, Nitro, Carboxy, Carbamoyl, durch C₁-C₄-Alkyl, C₁-C₄-Alkoxy, C₁-C₄-Alkylthio, C₁-C₄-Alkylsulfinyl, C₁-C₄-Alkylsulfonyl oder C₁-C₄-Alkoxycarbonyl (welche jeweils gegebenenfalls durch Halogen substituiert sind), durch Phenyl, Phenoxy oder Phenylthio (welche jeweils gegebenenfalls durch Halogen, Cyano, C₁-C₄-Alkyl, C₁-C₄-Halogenalkyl, C₁-C₄-Alkoxy und/oder C₁-C₄-Halogenalkoxy substituiert sind) substituiertes Heterocyclyl oder Heterocyclylalkyl mit 2 bis 6 Kohlenstoffatomen und 1 bis 4 Stickstoffatomen und/oder 1 bis 2 Sauerstoff- oder Schwefelatomen im gesättigten oder ungesättigten Heterocyclylteil und gegebenenfalls 1 bis 4 Kohlenstoffatomen im Alkylteil steht.

## Claims

1. Heterocyclylbenzonitriles of the general formula (I) 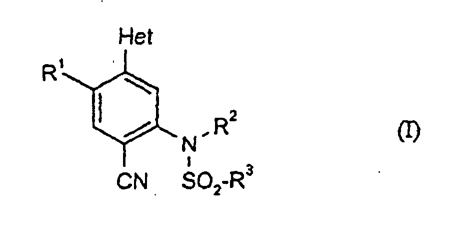 in which
R¹ represents hydrogen or halogen,
R² represents hydrogen, or represents formyl, or represents alkyl, alkenyl, alkinyl, alkoxy, alkylcarbonyl, alkoxycarbonyl or alkylsulphonyl having in each case up to 6 carbon atoms and in each case optionally substituted by halogen, cyano, C₁-C₄-alkoxy or C₁-C₄-alkoxy-carbonyl, or represents cycloalkyl, cycloalkylalkyl, cycloalkylcarbonyl or cycloalkylsulphonyl having 3 to 6 carbon atoms in the cycloalkyl part and, where appropriate, 1 to 4 carbon atoms in the alkyl part and in each case optionally substituted by halogen, cyano or C₁-C₄-alkyl, or represents phenylmethyl, phenylcarbonyl, naphthylcarbonyl, phenylmethylcarbonyl, phenoxycarbonyl, phenylsulphonyl, naphthylsulfonyl, phenylmethylsulphonyl, thienylsulphonyl, pyrazolylsulphonyl, pyridinylsulphonyl or pyridinylmethylsulphonyl (which are in each case optionally substituted by halogen, cyano, C₁-C₄-alkyl, C₁-C₄-halogenoalkyl, C₁-C₄-alkoxy, C₁-C₄-halogenoalkoxy or C₁-C₄-alkoxycarbonyl),
R³ represents alkyl, alkenyl or alkinyl having in each case up to 10 carbon atoms and in each case optionally substituted by halogen, cyano or C₁-C₄-alkoxy, or represents cycloalkyl or cycloalkylalkyl having 3 to 8 carbon atoms in the cycloalkyl part and, where appropriate, 1 to 4 carbon atoms in the alkyl part and in each case optionally substituted by halogen, cyano or C₁-C₄-alkyl, or represents aryl or arylalkyl having 6 or 10 carbon atoms in the aryl part and 1 to 4 carbon atoms in the alkyl part and in each case optionally substituted by halogen, cyano, nitro, carboxyl or carbamoyl, by C₁-C₄-alkyl, C₁-C₄-alkoxy, C₁-C₄-alkylthio, C₁-C₄-alkylsulphinyl or C₁-C₄-alkylsulphonyl (which are in each case optionally substituted by fluorine and/or chlorine), or by dimethylaminosulphonyl, diethylaminosulphonyl, dimethylaminocarbonyl or diethylaminocarbonyl, or by C₁-C₄-alkoxy-carbonyl (which is optionally substituted by halogen, methoxy or ethoxy), or by phenyl, phenyloxy or phenylthio (which are in each case optionally substituted by halogen, cyano, methyl, methoxy, trifluoromethyl and/or trifluoromethoxy), or represents heterocyclyl or heterocyclylalkyl having 2 to 6 carbon atoms and 1 to 4 nitrogen atoms and/or 1 to 2 oxygen or sulphur atoms in the saturated or unsaturated heterocyclyl part and, where appropriate, 1 to 4 carbon atoms in the alkyl part and in each case optionally substituted by halogen, cyano, nitro, carboxyl or carbamoyl, or by C₁-C₄-alkyl, C₁-C₄-alkoxy, C₁-C₄-alkylthio, C₁-C₄-alkylsulphinyl, C₁-C₄-alkylsulphonyl or C₁-C₄-alkoxycarbonyl (which are in each case optionally substituted by halogen) or by phenyl, phenoxy or phenylthio (which are in each case optionally substituted by halogen, cyano, C₁-C₄-alkyl, C₁-C₄-halogenoalkyl, C₁-C₄-alkoxy and/or C₁-C₄-halogenoalkoxy), and
Het represents one of the heterocyclic groupings listed below (bonded via N) 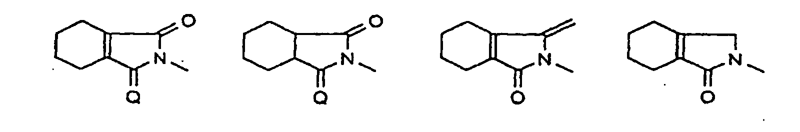 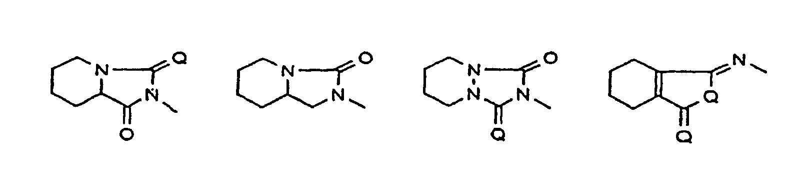 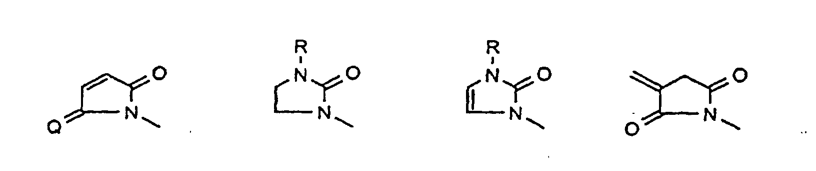 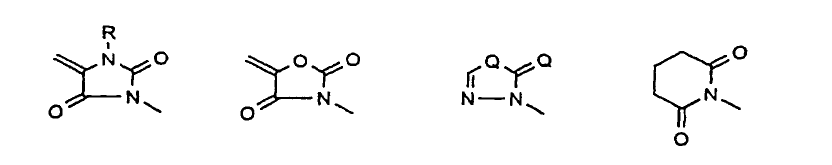 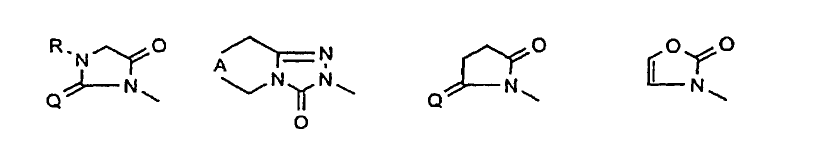 wherein, in each case, where appropriate,
A represents C₁-C₃-alkanediyl which is optionally interrupted by SO₂ and
Q represents oxygen or sulphur, and wherein the heterocyclic groupings mentioned are in each case optionally substituted once to three times by identical or different radicals from the series consisting of hydroxyl, halogen, cyano, nitro, C₁-C₄-alkyl, C₁-C₄-halogenoalkyl, C₁-C₄-alkoxy, C₁-C₄-halogenoalkoxy or phenyl and
R represents a radical from the series consisting of hydrogen, hydroxyl, cyano, nitro, C₁-C₄-alkyl, C₁-C₄-halogenoalkyl, C₁-C₄-alkoxy, C₁-C₄-halogenoalkoxy or phenyl.

2. Heterocyclylbenzonitriles according to Claim 1, **characterized in that**
R¹ represents hydrogen, fluorine or chlorine,
R² represents hydrogen, or represents formyl, or represents methyl, ethyl, nor i-propyl, n-, i-, s- or t-butyl, propenyl, butenyl, propinyl, butinyl, methoxy, ethoxy, n- or i-propoxy, n-, i- or s-butoxy, acetyl, propionyl, butyroyl, methoxycarbonyl, ethoxycarbonyl, n- or i-propoxycarbonyl, methylsulphonyl, ethylsulphonyl, n- or i-propylsulphonyl or n-, i-, s- or t-butylsulphonyl, in each case optionally substituted by fluorine, chlorine, cyano, methoxy, ethoxy, methoxycarbonyl or ethoxycarbonyl, or represents cyclopropyl, cyclobutyl, cyclopentyl, cyclohexyl, cyclopropylmethyl, cyclobutylmethyl, cyclopentylmethyl, cyclohexylmethyl, cyclopropylcarbonyl, cyclobutylcarbonyl, cyclopentylcarbonyl, cyclohexylcarbonyl, cyclopropylsulphonyl, cyclobutylsulphonyl, cyclopentylsulphonyl or cyclohexylsulphonyl, in each case optionally substituted by fluorine, chlorine, bromine, cyano, methyl, ethyl or n- or i-propyl, or represents phenylmethyl, phenylcarbonyl, phenylmethylcarbonyl, phenoxycarbonyl, phenylsulphonyl, phenylmethylsulphonyl, thienylsulphonyl, pyrazolylsulphonyl or pyridinylsulphonyl (which are in each case optionally substituted by fluorine, chlorine, bromine, cyano, methyl, ethyl, n- or i-propyl, n-, i-, s- or t-butyl, trifluoromethyl, methoxy, ethoxy, difluoromethoxy, trifluoromethoxy, methoxycarbonyl orethoxycarbonyl),
R³ represents methyl, ethyl, n- or i-propyl, n-, i-, s- or t-butyl, propenyl, butenyl, propinyl or butinyl, in each case optionally substituted by fluorine, chlorine, cyano, methoxy or ethoxy, or represents cyclopropyl, cyclobutyl, cyclopentyl, cyclohexyl, cyclopropylmethyl, cyclobutylmethyl, cyclopentylmethyl or cyclohexylmethyl, in each case optionally substituted by fluorine, chlorine, bromine, cyano, methyl, ethyl or n- or i-propyl, or represents phenyl or phenylmethyl, in each case optionally substituted by fluorine, chlorine, bromine, cyano, nitro or carboxyl, or by methyl, ethyl, n- or i-propyl, n-, i-, s- or t-butyl, methoxy, ethoxy, methylthio, ethylthio, methylsulphinyl, ethylsulphinyl, methylsulphonyl or ethylsulphonyl (which are in each case optionally substituted by fluorine and/or chlorine), or by dimethylaminosulphonyl or dimethylaminocarbonyl, or by methoxycarbonyl orethoxycarbonyl (which are in each case optionally substituted by fluorine, chlorine, methoxy or ethoxy), or by phenyl or phenoxy, or represents thienyl, pyrazolyl, pyridinyl or pyridinylmethyl, in each case optionally substituted by fluorine, chlorine, bromine, cyano or nitro, or by methyl, ethyl, n- or i-propyl, n-, i-, s- or t-butyl, methoxy, ethoxy, n- or i-propoxy, methylthio, ethylthio, methylsulphinyl, ethylsulphinyl, methylsulphonyl, ethylsulphonyl, methoxycarbonyl or ethoxycarbonyl (which are in each case optionally substituted by fluorine and/or chlorine), or by phenyl or phenoxy, and
A represents methylene, dimethylene or trimethylene,
Q represents oxygen, and wherein the heterocyclic groupings mentioned are in each case optionally substituted once or twice by identical or different radicals from the series consisting of hydroxyl, fluorine, chlorine, bromine, cyano, nitro, methyl, ethyl, n- or i-propyl, trifluoromethyl, methoxy, ethoxy, n- or i-propoxy, difluoromethoxy, trifluoromethoxy or phenyl and
R represents a radical from the series consisting of hydrogen, hydroxyl, cyano, nitro, C₁-C₄-alkyl, C₁-C₄-halogenoalkyl, C₁-C₄-alkoxy, C₁-C₄-halogenoalkoxy or phenyl.

3. Process for the preparation of heterocyclylbenzonitriles according to Claim 1 or 2, **characterized in that**
(a) anhydrides of the general formulae (IIa) to (IIg) 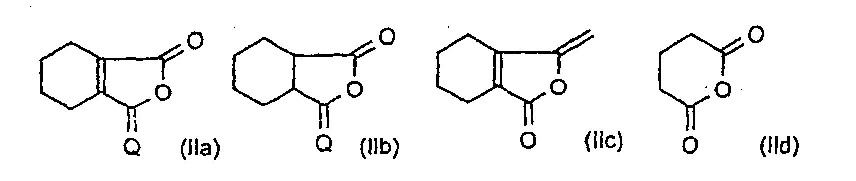 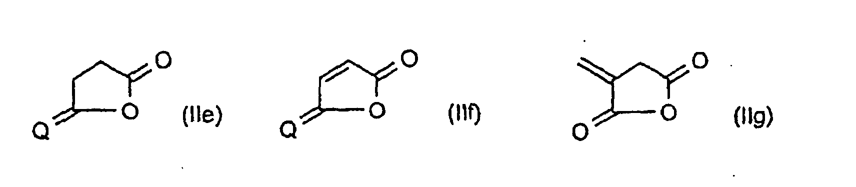 in which
Q has the meaning given in Claim 1 or 2, are reacted with aminobenzonitriles of the general formula (III) 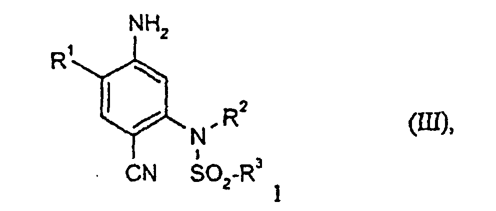 in which
R¹, R² and R³ have the meanings given in Claim 1 or 2,
if appropriate in the presence of a diluent and if appropriate in the presence of a reaction auxiliary,
or **in that**
(b) halogenated heterocyclylbenzonitriles of the general formula (IV) 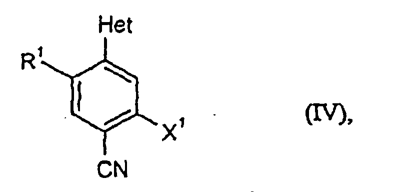 in which
Het and R¹ have the meanings given in Claim 1 or 2 and
X¹ represents halogen,
are reacted with sulphonamides of the general formula (V) 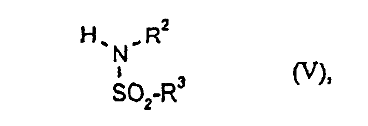 in which
R² and R³ have the meanings given in Claim 1 or 2,
if appropriate in the presence of a diluent and if appropriate in the presence of an acid acceptor.

4. Method of combating unwanted plants, **characterized in that** heterocyclylbenzonitriles according to Claim 1 or 2 are allowed to act on unwanted plants and/or their environment.

5. Use of heterocyclylbenzonitriles according to Claim 1 or 2 for combating unwanted plants.

6. Process for the preparation of herbicidal compositions, **characterized in that** heterocyclylbenzonitriles according to Claim 1 or 2 are mixed with extenders and/or surface-active substances.

7. Herbicidal compositions, **characterized by** a content of at least one heterocyclylbenzonitrile according to Claim 1 or 2.

8. Aminobenzonitriles of the general formula (III) 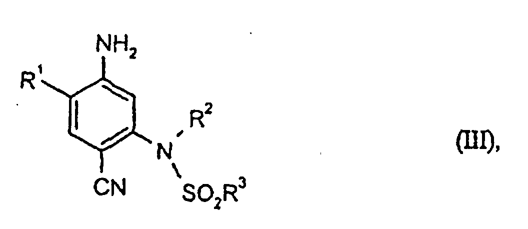 in which
R¹ represents hydrogen or halogen,
R² represents formyl, or represents alkenyl, alkinyl, alkoxy, alkylcarbonyl, alkoxycarbonyl or alkylsulphonyl having in each case up to 6 carbon atoms and in each case optionally substituted by halogen, cyano, C₁-C₄-alkoxy or C₁-C₄-alkoxy-carbonyl, or represents cycloalkyl, cycloalkylalkyl, cycloalkylcarbonyl or cycloalkylsulphonyl having 3 to 6 carbon atoms in the cycloalkyl part and, where appropriate, 1 to 4 carbon atoms in the alkyl part and in each case optionally substituted by halogen, cyano or C₁-C₄-alkyl, or represents phenylmethyl, phenylcarbonyl, naphthylcarbonyl, phenylmethylcarbonyl, phenoxycarbonyl, phenylsulphonyl, naphthylsulfonyl, phenylmethylsulphonyl, thienylsulphonyl, pyrazolylsulphonyl, pyridinylsulphonyl or pyridinylmethylsulphonyl (which are in each case optionally substituted by halogen, cyano, C₁-C₄-alkyl, C₁-C₄-halogenoalkyl, C₁-C₄-alkoxy, C₁-C₄-halogenoalkoxy or C₁-C₄-alkoxycarbonyl), and
R³ represents alkyl, alkenyl or alkinyl having in each case up to 10 carbon atoms and in each case optionally substituted by halogen, cyano or C₁-C₄-alkoxy, or represents cycloalkyl or cycloalkylalkyl having 3 to 8 carbon atoms in the cycloalkyl part and, where appropriate, 1 to 4 carbon atoms in the alkyl part and in each case optionally substituted by halogen, cyano or C₁-C₄-alkyl, or represents aryl or arylalkyl having 6 or 10 carbon atoms in the aryl part and 1 to 4 carbon atoms in the alkyl part and in each case optionally substituted by halogen, cyano, nitro, carboxyl or carbamoyl, by C₁-C₄-alkyl, C₁-C₄-alkoxy, C₁-C₄-alkylthio, C₁-C₄-alkylsulphinyl or C₁-C₄-alkylsulphonyl (which are in each case optionally substituted by fluorine and/or chlorine), or by dimethylaminosulphonyl, diethylaminosulphonyl, dimethylaminocarbonyl or diethylaminocarbonyl, or by C₁-C₄-alkoxy-carbonyl (which is optionally substituted by halogen, methoxy or ethoxy), or by phenyl, phenyloxy or phenylthio (which are in each case optionally substituted by halogen, cyano, methyl, methoxy, trifluoromethyl and/or trifluoromethoxy), or represents heterocyclyl or heterocyclylalkyl having 2 to 6 carbon atoms and 1 to 4 nitrogen atoms and/or 1 to 2 oxygen or sulphur atoms in the saturated or unsaturated heterocyclyl part and, where appropriate, 1 to 4 carbon atoms in the alkyl part and in each case optionally substituted by halogen, cyano, nitro, carboxyl or carbamoyl, or by C₁-C₄-alkyl, C₁-C₄-alkoxy, C₁-C₄-alkylthio, C₁-C₄-alkylsulphinyl, C₁-C₄-alkylsulphonyl or C₁-C₄-alkoxycarbonyl (which are in each case optionally substituted by halogen) or by phenyl, phenoxy or phenylthio (which are in each case optionally substituted by halogen, cyano, C₁-C₄-alkyl, C₁-C₄-halogenoalkyl, C₁-C₄-alkoxy and/or C₁-C₄-halogenoalkoxy).

## Revendications

1. Hétérocyclylbenzonitriles répondant à la formule générale (I) 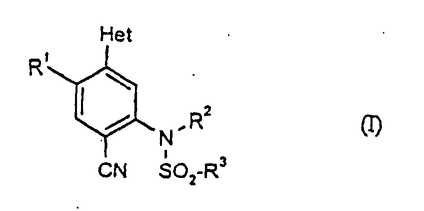 dans laquelle
R¹ représente un atome d'hydrogène ou un atome d'halogène,
R² représente un atome d'hydrogène; un groupe formyle; ou représente un groupe alkyle, un groupe alcényle, un groupe alcynyle, un groupe alcoxy, un groupe alkylcarbonyle, un groupe alcoxycarbonyle ou un groupe alkylsulfonyle, contenant chacun jusqu'à 6 atomes de carbone, chacun de ces groupes portant le cas échéant un ou plusieurs substituants identiques ou différents halogéno, cyano, alcoxy en C₁-C₄ ou alcoxy(en C₁-C₄)carbonyle; ou représente un groupe cycloalkyle, un groupe cycloalkylalkyle, un groupe cycloalkylcarbonyle ou un groupe cycloalkylsulfonyle, contenant de 3 à 6 atomes de carbone dans la fraction cycloalkyle et le cas échéant de 1 à 4 atomes de carbone dans la fraction alkyle, chacun de ces groupes portant le cas échéant un ou plusieurs substituants identiques ou différents halogéno, cyano ou alkyle en C₁-C₄; ou représente un groupe phénylméthyle, un groupe phénylcarbonyle, un groupe naphtylcarbonyle, un groupe phényl-méthylcarbonyle, un groupe phénoxycarbonyle, un groupe phénylsulfonyle, un groupe naphtyl-sulfonyle, un groupe phénylméthylsulfonyle, un groupe thiénylsulfonyle, un groupe pyrazolylsulfonyle, un groupe pyridinylsulfonyle ou un groupe pyridinylméthylsulfonyle (chacun de ces groupes portant le cas échéant un ou plusieurs substituants identiques ou différents halogéno, cyano, alkyle en C₁-C₄, halogénalkyle en C₁-C₄, alcoxy en C₁-C₄, halogénalcoxy en C₁-C₄ ou alcoxy(en C₁-C₄)-carbonyle),
R³ représente un groupe alkyle, un groupe alcényle ou un groupe alcynyle contenant chacun jusqu'à 10 atomes de carbone, chacun de ces groupes portant le cas échéant un ou plusieurs substituants identiques ou différents halogéno, cyano, ou alcoxy en C₁-C₄; ou représente un groupe cycloalkyle ou un groupe cycloalkylalkyle contenant de 3 à 8 atomes de carbone dans la fraction cycloalkyle et le cas échéant de 1 à 4 atomes de carbone dans la fraction alkyle, chacun de ces groupes portant le cas échéant un ou plusieurs substituants identiques ou différents halogéno, cyano ou alkyle en C₁-C₄; ou représente un groupe aryle ou un groupe arylalkyle contenant 6 ou 10 atomes de carbone dans la fraction aryle et de 1 à 4 atomes de carbone dans la fraction alkyle, chacun de ces groupes portant le cas échéant un ou plusieurs substituants identiques ou différents halogéno, cyano, nitro, carboxyle, carbamoyle; alkyle en C₁-C₄, alcoxy en C₁-C₄, alkyl(en C₁C₄)thio, alkyl(en C₁-C₄)sulfinyle ou alkyl(en C₁-C₄)sulfonyle (chacun de ces groupes portant le cas échéant un ou plusieurs substituants fluoro et/ou chloro); diméthylaminosulfonyle, diéthylaminosulfonyle, diméthylaminocarbonyle ou diéthylaminocarbonyle; alcoxy(en C₁-C₄)-carbonyle (portant le cas échéant un ou plusieurs substituants halogéno, méthoxy ou éthoxy); phényle, phénoxy ou phénylthio (chacun de ces groupes portant le cas échéant un ou plusieurs substituants identiques ou différents halogéno, cyano, méthyle, méthoxy, trifluorométhyle et/ou trifluorométhoxy); ou représente un groupe hétérocyclyle ou un groupe hétérocyclylalkyle contenant de 2 à 6 atomes de carbone et de 1 à 4 atomes d'azote et/ou de 1 à 2 atomes d'oxygène ou de soufre dans la fraction hétérocyclyle saturée ou insaturée et le cas échéant de 1 à 4 atomes de carbone dans la fraction alkyle, chacun de ces groupes portant le cas échéant un ou plusieurs substituants identiques ou différents halogéno, cyano, nitro, carboxyle, carbamoyle; alkyle en C₁-C₄, alcoxy en C₁-C₄, alkyl(en C₁-C₄)thio, alkyl(en C₁-C₄)sulfinyle, alkyl(en C₁-C₄)sulfonyle ou alkyl(en C₁-C₄)carbonyle (chacun de ces groupes portant le cas échéant un ou plusieurs substituants halogéno identiques ou différents); phényle, phénoxy ou phénylthio (chacun de ces groupes portant le cas échéant un ou plusieurs substituants halogéno, cyano, alkyle en C₁-C₄, halogénalkyle en C₁-C₄, alcoxy en C₁-C₄ et/ou halogénalcoxy en C₁-C₄), et
Het représente un des groupements hétérocycliques (reliés via l'atome d'azote) indiqués ci-après 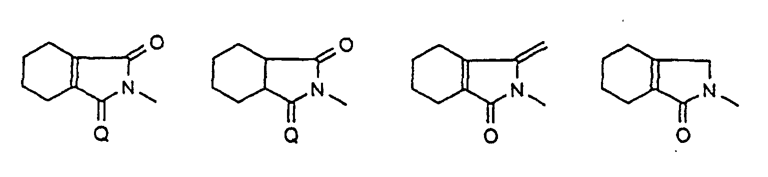 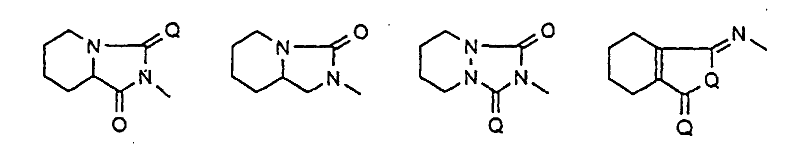 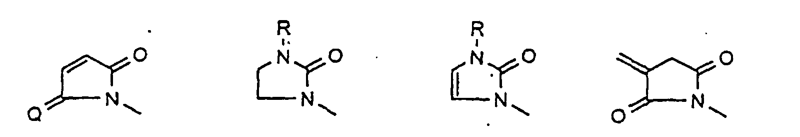 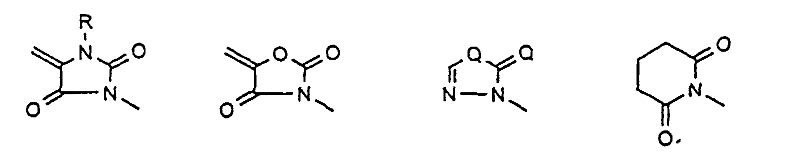 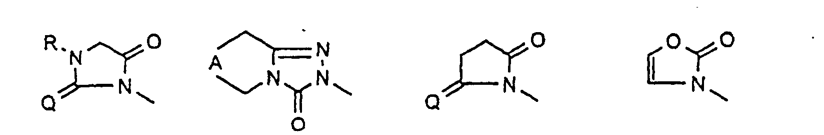 dans lesquels, dans chacun de ces groupements, le cas échéant,
A représente un groupe alcanediyle en C₁-C₃ le cas échéant interrompu par un groupe SO₂, et
Q représente un atome d'oxygène ou un atome de soufre, et dans lesquels les groupements hétérocycliques indiqués portent le cas échéant de 1 à 3 substituants identiques ou différents choisis parmi la série de radicaux comprenant un groupe hydroxyle, un atome d'halogène, un groupe cyano, un groupe nitro, un groupe alkyle en C₁-C₄, un groupe halogénalkyle en C₁-C₄, un groupe alcoxy en C₁-C₄, un groupe halogénalcoxy en C₁-C₄ ou un groupe phényle, et
R représente un radical choisi parmi la série comprenant un atome d'hydrogène, un groupe hydroxyle, un groupe cyano, un groupe nitro, un groupe alkyle en C₁-C₄, un groupe halogénalkyle en C₁-C₄, un groupe alcoxy en C₁-C₄, un groupe halogénalcoxy en C₁-C₄ ou un groupe phényle.

2. Hétérocyclylbenzonitriles selon la revendication 1, **caractérisés en ce que**
R¹ représente un atome d'hydrogène, un atome de fluor ou un atome de chlore,
R² représente un atome d'hydrogène; un groupe formyle; ou représente un groupe méthyle, un groupe éthyle, un groupe n- ou i-propyle, un groupe n-, i-, s- ou t-butyle, un groupe propényle, un groupe butényle, un groupe propynyle, un groupe butynyle, un groupe méthoxy, un groupe éthoxy, un groupe n- ou i-propoxy, un groupe n-, i- ou s-butoxy, un groupe acétyle, un groupe propionyle, un groupe butyryle, un groupe méthoxycarbonyle, un groupe éthoxycarbonyle, un groupe n- ou i-propoxycarbonyle, un groupe méthylsulfonyle, un groupe éthylsulfonyle, un groupe n- ou i-propylsulfonyle, un groupe n-, i-, s- ou t-butylsulfonyle, chacun de ces groupes portant le cas échéant un ou plusieurs substituants identiques ou différents fluoro, chloro, cyano, méthoxy, éthoxy, méthoxycarbonyle ou éthoxycarbonyle; ou représente un groupe cyclopropyle, un groupe cyclobutyle, un groupe cyclopentyle, un groupe cyclohexyle, un groupe cyclopropylméthyle, un groupe cyclobutylméthyle, un groupe cyclopentylméthyle, un groupe cyclohexylméthyle, un groupe cyclopropylcarbonyle, un groupe cyclobutylcarbonyle, un groupe cyclopentylcarbonyle, un groupe cyclohexylcarbonyle, un groupe cyclopropylsulfonyle, un groupe cyclobutylsulfonyle, un groupe cyclopentylsulfonyle ou un groupe cyclohexylsulfonyle, chacun de ces groupes portant le cas échéant un ou plusieurs substituants identiques ou différents fluoro, chloro, bromo, cyano, méthyle, éthyle, n- ou i-propyle; ou représente un groupe phénylméthyle, un groupe phénylcarbonyle, un groupe phénylméthylcarbonyle, un groupe phénoxycarbonyle, un groupe phénylsulfonyle, un groupe phénylméthylsulfonyle, un groupe thiénylsulfonyle, un groupe pyrazolylsulfonyle ou un groupe pyridinylsulfonyle (chacun de ces groupes portant le cas échéant un ou plusieurs substituants identiques ou différents fluoro, chloro, bromo, cyano, méthyle, éthyle, n- ou i-propyle, n-, i-, s- ou t-butyle, trifluorométhyle, méthoxy, éthoxy, difluorométhoxy, trifluorométhoxy, méthoxycarbonyle ou éthoxycarbonyle),
R³ représente un groupe méthyle, un groupe éthyle, un groupe n- ou i-propyle, un groupe n-, i-, s- ou t-butyle, un groupe propényle, un groupe butényle, un groupe propynyle, un groupe butynyle, chacun de ces groupes portant le cas échéant un ou plusieurs substituants identiques ou différents fluoro, chloro, cyano, méthoxy, éthoxy; ou représente un groupe cyclopropyle, un groupe cyclobutyle, un groupe cyclopentyle, un groupe cyclohexyle, un groupe cyclopropylméthyle, un groupe cyclobutylméthyle, un groupe cyclopentylméthyle ou un groupe cyclohexylméthyle, chacun de ces groupes portant le cas échéant un ou plusieurs substituants identiques ou différents fluoro, chloro, bromo, cyano, méthyle, éthyle, n- ou i-propyle; ou représente un groupe phényle ou un groupe phénylméthyle, chacun de ces groupes portant le cas échéant un ou plusieurs substituants identiques ou différents fluoro, chloro, bromo, cyano, nitro, carboxyle; méthyle, éthyle, n- ou i-propyle, n-, i-, s- ou t-butyle, méthoxy, éthoxy, méthylthio, éthylthio, méthylsulfinyle, éthylsulfinyle, méthylsulfonyle, éthylsulfonyle (chacun de ces groupes portant le cas échéant un ou plusieurs substituants fluoro et/ou chloro); diméthylaminosulfonyle ou diméthylamino-carbonyle; méthoxycarbonyle ou éthoxycarbonyle (chacun de ces groupes portant le cas échéant un ou plusieurs substituants identiques ou différents fluoro, chloro, méthoxy ou éthoxy); ou représente un groupe thiényle, un groupe pyrazolyle, un groupe pyridinyle ou un groupe pyridinylméthyle, chacun de ces groupes portant le cas échéant un ou plusieurs substituants identiques ou différents fluoro, chloro, bromo, cyano, nitro; méthyle, éthyle, n- ou i-propyle, n-, i-, s- ou t-butyle, méthoxy, éthoxy, n- ou i-propoxy, méthylthio, éthylthio, méthyl-sulfinyle, éthylsulfinyle, méthylsulfonyle, éthylsulfonyle, méthoxy-carbonyle ou éthoxycarbonyle (chacun de ces groupes portant le cas échéant un ou plusieurs substituants fluoro et/ou chloro) phényle ou phénoxy; et
A représente un groupe méthylène, un groupe diméthylène ou un groupe triméthylène;
Q représente un atome d'oxygène et dans lesquels les groupements hétérocycliques indiqués portent le cas échéant 1 ou 2 substituants identiques ou différents choisis parmi la série de radicaux comprenant un groupe hydroxyle, un atome de fluor, un atome de chlore, un atome de brome, un groupe cyano, un groupe nitro, un groupe méthyle, un groupe éthyle, un groupe n- ou i-propyle, un groupe trifluorométhyle, un groupe méthoxy, un groupe éthoxy, un groupe n- ou i-propoxy, un groupe difluorométhoxy, un groupe trifluorométhoxy ou un groupe phényle, et
R représente un radical choisi parmi la série comprenant un atome d'hydrogène, un groupe hydroxyle, un groupe cyano, un groupe nitro, un groupe alkyle en C₁-C₄, un groupe halogénalkyle en C₁-C₄, un groupe alcoxy en C₁-C₄, un groupe halogénalcoxy en C₁-C₄ ou un groupe phényle.

3. Procédé pour la préparation d'hétérocyclylbenzonitriles selon la revendication 1 ou 2, **caractérisé en ce que**
(a) on fait réagir des anhydrides répondant aux formules générales (IIa) à (IIg), 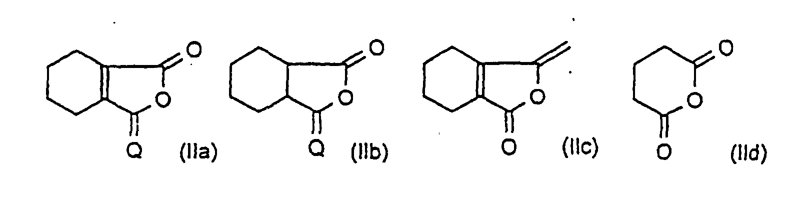 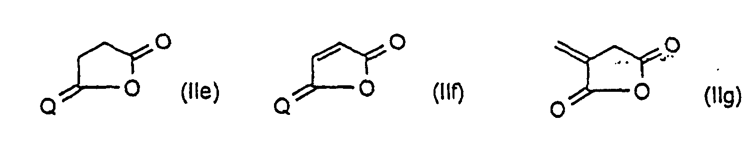 dans lesquelles
Q a la signification indiquée à la revendication 1 ou 2,
avec des aminobenzonitriles répondant à la formule générale (III) 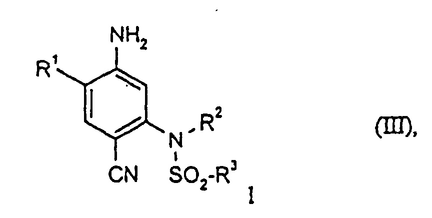 dans laquelle
R¹, R² et R³ ont les significations indiquées à la revendication 1 ou 2,
le cas échéant en présence d'un diluant et le cas échéant en présence d'un adjuvant réactionnel,
ou **en ce que**
(b) on fait réagir des hétérocyclylbenzonitriles halogénés répondant à la formule générale (IV) 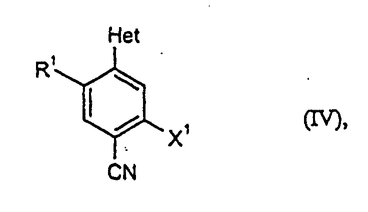 dans laquelle
Het et R¹ ont les significations indiquées à la revendication 1 ou 2, et
X¹ représente un atome d'halogène,
avec des sulfonamides répondant à la formule générale (V) 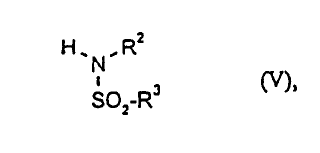 dans laquelle
R² et R³ ont les significations indiquées à la revendication 1 ou 2,
le cas échéant en présence d'un diluant et le cas échéant en présence d'un agent neutralisant les acides.

4. Procédé pour lutter contre des plantes indésirables, **caractérisé en ce qu'**on laisse agir des hétérocyclylbenzonitriles selon la revendication 1 ou 2 sur des plantes indésirables et/ou sur leur biotope.

5. Utilisation d'hétérocyclylbenzonitriles selon la revendication 1 ou 2 pour lutter contre des plantes indésirables.

6. Procédé pour la préparation d'agents herbicides, **caractérisé en ce qu'**on mélange des hétérocyclylbenzonitriles selon la revendication 1 ou 2 avec des diluants et/ou avec des substances tensioactives.

7. Agents herbicides **caractérisés par** une teneur en au moins un hétérocyclylbenzonitrile selon la revendication 1 ou 2.

8. Aminobenzonitriles répondant à la formule générale (III) 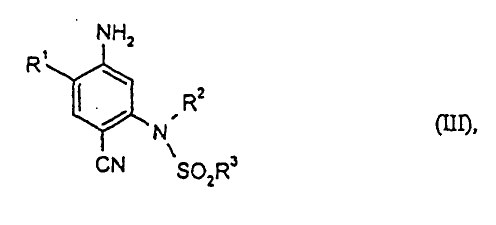 dans laquelle
R¹ représente un atome d'hydrogène ou un atome d'halogène,
R² représente un groupe formyle; ou représente un groupe alcényle, un groupe alcynyle, un groupe alcoxy, un groupe alkylcarbonyle, un groupe alcoxycarbonyle ou un groupe alkylsulfonyle, contenant chacun jusqu'à 6 atomes de carbone, chacun de ces groupes portant le cas échéant un ou plusieurs substituants identiques ou différents halogéno, cyano, alcoxy en C₁-C₄ ou alcoxy(en C₁-C₄)carbonyle; ou représente un groupe cycloalkyle, un groupe cycloalkylalkyle, un groupe cycloalkylcarbonyle ou un groupe cycloalkylsulfonyle, contenant de 3 à 6 atomes de carbone dans la fraction cycloalkyle et le cas échéant de 1 à 4 atomes de carbone dans la fraction alkyle, chacun de ces groupes portant le cas échéant un ou plusieurs substituants identiques ou différents halogéno, cyano ou alkyle en C₁-C₄; ou représente un groupe phénylméthyle, un groupe phénylcarbonyle, un groupe naphtylcarbonyle, un groupe phénylméthylcarbonyle, un groupe phénoxycarbonyle, un groupe phénylsulfonyle, un groupe naphtylsulfonyle, un groupe phénylméthylsulfonyle, un groupe thiénylsulfonyle, un groupe pyrazolylsulfonyle, un groupe pyridinylsulfonyle ou un groupe pyridinylméthylsulfonyle (chacun de ces groupes portant le cas échéant un ou plusieurs substituants identiques ou différents halogéno, cyano, alkyle en C₁-C₄, halogénalkyle en C₁-C₄, alcoxy en C₁-C₄, halogénalcoxy en C₁-C₄ ou alcoxy(en C₁-C₄)-carbonyle), et
R³ représente un groupe alkyle, un groupe alcényle ou un groupe alcynyle contenant chacun jusqu'à 10 atomes de carbone, chacun de ces groupes portant le cas échéant un ou plusieurs substituants identiques ou différents halogéno, cyano, ou alcoxy en C₁-C₄; ou représente un groupe cycloalkyle ou un groupe cycloalkylalkyle contenant de 3 à 8 atomes de carbone dans la fraction cycloalkyle et le cas échéant de 1 à 4 atomes de carbone dans la fraction alkyle, chacun de ces groupes portant le cas échéant un ou plusieurs substituants identiques ou différents halogéno, cyano ou alkyle en C₁-C₄; ou représente un groupe aryle ou un groupe arylalkyle contenant 6 ou 10 atomes de carbone dans la fraction aryle et de 1 à 4 atomes de carbone dans la fraction alkyle, chacun de ces groupes portant le cas échéant un ou plusieurs substituants identiques ou différents halogéno, cyano, nitro, carboxyle, carbamoyle; alkyle en C₁-C₄, alcoxy en C₁-C₄, alkyl(en C₁-C₄)thio, alkyl(en C₁-C₄)sulfinyle ou alkyl(en C₁-C₄)sulfonyle (chacun de ces groupes portant le cas échéant un ou plusieurs substituants fluoro et/ou chloro); diméthylaminosulfonyle, diéthylaminosulfonyle, diméthylaminocarbonyle ou diéthylaminocarbonyle; alcoxy(en C₁-C₄)-carbonyle (portant le cas échéant un ou plusieurs substituants halogéno, méthoxy ou éthoxy); phényle, phénoxy ou phénylthio (chacun de ces groupes portant le cas échéant un ou plusieurs substituants halogéno, cyano, méthyle, méthoxy, trifluorométhyle et/ou trifluorométhoxy); ou représente un groupe hétérocyclyle ou un groupe hétérocyclylalkyle contenant de 2 à 6 atomes de carbone et de 1 à 4 atomes d'azote et/ou de 1 à 2 atomes d'oxygène ou de soufre dans la fraction hétérocyclyle saturée ou insaturée et le cas échéant de 1 à 4 atomes de carbone dans la fraction alkyle, chacun de ces groupes portant le cas échéant un ou plusieurs substituants identiques ou différents halogéno, cyano, nitro, carboxyle, carbamoyle; alkyle en C₁-C₄, alcoxy en C₁-C₄, alkyl(en C₁-C₄)thio, alkyl(en C₁-C₄)sulfinyle, alkyl(en C₁-C₄)sulfonyle ou alkyl(en C₁-C₄)carbonyle (chacun de ces groupes portant le cas échéant un ou plusieurs substituants halogéno identiques ou différents); phényle, phénoxy ou phénylthio (chacun de ces groupes portant le cas échéant un ou plusieurs substituants halogéno, cyano, alkyle en C₁-C₄, halogénalkyle en C₁-C₄, alcoxy en C₁-C₄ et/ou halogénalcoxy en C₁-C₄).
